(19) Europäisches Patentamt
European Patent Office
Office européen des brevets

(11) EP 2 620 211 A2

(12) # EUROPEAN PATENT APPLICATION

(43) Date of publication:
**31.07.2013 Bulletin 2013/31**

(51) Int Cl.:
***B01J 13/18*** *(2006.01)* ***C11D 3/50*** *(2006.01)*
***B01J 13/14*** *(2006.01)*

(21) Application number: **13305075.7**

(22) Date of filing: **23.01.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.01.2012 EP 12305080**

(71) Applicant: **Takasago International Corporation**
**Tokyo 144-8721 (JP)**

(72) Inventors:
- **Warr, Jonathan**
  **75017 PARIS (FR)**
- **Ribaut, Tiphaine**
  **75017 PARIS (FR)**
- **Aussant, Emmanuel**
  **75017 PARIS (FR)**
- **Antony, Olivier**
  **75017 PARIS (FR)**
- **Fraser, Stuart**
  **Little Neston, Cheshire CH64 4DH (GB)**

(74) Representative: **Brunetti, Fabrizio et al**
**Cabinet Beau de Loménie**
**158, rue de l'Université**
**75340 Paris Cedex 07 (FR)**

(54) **New microcapsules**

(57) The present invention relates to a novel microcapsule which is endowed with reduced leakage of encapsulated materials. The microcapsule contains one or more fragrances and is suitable for inclusion in non-edible consumer goods products, laundry products, personal care products and cosmetic products. The microcapsule can be obtained by polymerizing an emulsion so that emulsion droplets are encapsulated into a subsequently cured polymeric shell.

EP 2 620 211 A2

## Description

### Field of the Invention

The invention relates to a microcapsule having a particular average particle size and which comprises a fragrance-containing core and a polymeric shell enclosing said core, a process for the manufacture of that microcapsule, non-ingestible consumer products (such as household cleaners, laundry products, personal care products and cosmetic products) containing that microcapsule and the use of particular polymer crosslinkers to reduce the leakage from microcapsules comprising a fragrance-containing core and a polymeric shell enclosing said core.

### Background of the Invention

The use of encapsulation technology to protect ingredients such as perfume, pesticides and pharmaceutical agents during storage and to control delivery is well known in many industries. Capsules based condensation polymers containing urea, melamine and formaldehyde are used commercially in laundry and cosmetic to control fragrance delivery and performance. However there are concerns about the presence of formaldehyde in the final product composition since formaldehyde is considered to have carcinogenic properties. Experience has also shown these capsules to have other drawbacks such as the core ingredients leaking during storage.

WO9924525 discloses latent heat storage media comprising certain microcapsules for use in the construction industry. WO9924525 microcapsules have a preferred average particle size of 3 to 12 microns and only one size of 4.22 microns is exemplified. Additionally, WO9924525 is silent as to the problem of microcapsule leakage and discloses a list of several, apparently equally suitable, alternative crosslinkers whereas no mention is made of advantages following the adoption of non di- or poly-acrylate crosslinkers. Also, all examples of WO9924525 contain a generic butanediol diacrylate as crosslinker but no mention is made of the diol position thereof.

WO2005116559 discloses moldings of lignocellulose materials and a glue resin, a process for producing them and a binder composition comprising glue resin and microcapsules for use in the construction industry. Like WO9924525, also WO2005116559 is silent as to the problem of microcapsule leakage and discloses a list of several, apparently equally suitable, alternative crosslinkers whereas no mention is made of advantages following the adoption of non di- or polyacrylates crosslinkers. Also, all examples of WO2005116559 contain a generic butanediol diacrylate as crosslinker but no mention is made of the diol position thereof.

WO2005105291 discloses core shell particles that comprise a substantially impervious shell wall. WO2005105291 particle shell comprises a copolymer formed inter alia from 5 to 90% by weight of a multifunctional monomer. As to multifunctional monomers, preference is given to di- and polyacrylates where the highly preferred one is 1,4-butanediol diacrylate and no mention is made of advantages following the adoption of non di- or polyacrylates crosslinkers. All examples of WO2005105291 contain a generic butanediol diacrylate but no mention is made of the diol position thereof.

US6849591 discloses microcapsule formulations, laundry detergent and cleaning product compositions comprising microcapsules, said microcapsules containing in their core a hydrophobic material and, in particular, a fragrance or perfume. In certain embodiments, US6849591 microcapsule shell is comprised of from 1 to 100% by weight, of an anionogenic monoethylenically unsaturated monomers and/or polyethylenically unsaturated monomers whose unsaturated sites are connected via successive chemical bonds of which at least one bond is base-hydrolysable; or cationogenic monoethylenically unsaturated monomers and/or polyethylenically unsaturated monomers whose unsaturated sites are connected via successive chemical bonds of which at least one bond is acid-hydrolysable; from 0 to 95% by weight of neutral monoethylenically unsaturated monomers, from 0 to 80% by weight of monomers having a permanent crosslinking action, containing at least two ethylenically unconjugated double bonds per molecule, and from 0 to 20% by weight of water-soluble monoethylenically unsaturated monomers, the amounts of the monomers adding up to 100% by weight. Few specific examples of polyethylenically unsaturated monomers are provided in US6849591 and the document is totally silent as to the conditions (e.g. temperature or time) in which acid- or base-hydrolysability is to be measured or the extent (total vs. partial) of monomer hydrolysis. Additionally US6849591 discloses a list of several, but apparently equally suitable, alternative monomers having a permanent crosslinking action where however none of the examples discloses compositions containing a monomer having a permanent crosslinking action. Similarly no mention is made of advantages following the adoption of non di- or polyacrylate crosslinkers.

US6951836 discloses microcapsule formulations, laundry detergent and cleaning product compositions comprising microcapsules containing a fragrance or perfume in their core. US6951836 discloses a list of several, but apparently equally suitable, alternative bi- or polyfunctional monomers. US6951836 does not exemplify any particular composition and no mention is made in it of advantages following the adoption of non di- or polyacrylates crosslinkers.

WO2010119020 discloses a carrier system for fragrances, the production thereof and the use of the carrier system in various technical areas. All examples of WO2010119020, notably examples 10 and 11 which are said to be

the most performing ones, contain 1,4-butanediol diacrylate in significant amounts. No mention is made of non-acrylate derivatives but ethylene glycol dimethacrylate or advantages following the adoption of such non-acrylate derivatives. Finally, microcapsules exemplified in WO2010119020 have a diameter comprised between 2.13 and 5.92 microns.

US20100286018 discloses certain microcapsules obtainable from several, but apparently equally suitable, alternative monomers having a permanent crosslinking action. All of the examples of US20100286018 contain di- or polyacrylates derivatives (in particular 1,4-butanediol diacrylate) and no mention is made of advantages following the adoption of non-acrylate crosslinkers. Finally, microcapsules exemplified in US20100286018 have a diameter comprised between 2.179 and 5.567 microns.

Accordingly, there is still the need for microcapsules having an appropriate dimension to allow one to incorporate sufficient amounts of fragrances without being visible to naked eye when deposited on a black surface, showing a reduced leakage notably upon storage and preferably in liquid media, and which may allow one to avoid the use of formaldehyde. This need is particularly keen in the field of non-ingestible consumer products (such as household cleaners, laundry products, personal care products and cosmetic products), especially those including a liquid medium, which has quite different technical constraints from other fields such as the construction industry or fabric coating.

## Summary of the Invention

It is thus an object of the present invention to provide a microcapsule comprising a fragrance-containing core and a polymeric shell enclosing said core which is endowed with a reduced leakage of the material comprised in the core upon storage, especially when the microcapsule is dispersed in a liquid medium and used in the context of e.g. a non-edible consumer goods product, a laundry product, a personal care product or a cosmetic product. It is another object of the invention to provide a microcapsule as defined above which is free from formaldehyde. It is another object of the invention to provide a simple and effective process for the manufacture of a microcapsule as defined above. It is another object of the present invention to provide a technical solution to reduce leakage from a fragrance-containing microcapsule such as the one defined above especially when the microcapsule is part of a non-ingestible consumer product, more especially liquid consumer products such as household cleaners, laundry products, personal care products, and cosmetic products.

In one aspect, the present invention relates to a microcapsule having an average particle size comprised between 7.5 and 50 microns, said capsule comprising a core and a polymeric shell enclosing said core, wherein:

the core comprises a core material comprising an emulsifiable fragrance, and
the shell comprises in polymerized form a monomer blend comprising:

i) a monomer (I) selected from the group consisting of:

- a monoethylenically unsatured monomer,
- dimethyldiallyl ammonium chloride (DMDAAC), and
- mixtures thereof
  and

ii) a monomer (II) which is a polyethylenically unsatured monomer and which comprises one or more monomers selected from the group consisting of divinylbenzene, trivinylbenzene, and a methacrylic ester derived from polyhydric linear or branched $C_2$-$C_{24}$ alcohols or from linear or branched $C_2$-$C_{24}$ polyethylene glycols,
wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.

In one aspect, the present invention relates to a microcapsule having an average particle size comprised between 7.5 and 50 microns, said capsule comprising a core and a polymeric shell enclosing said core, wherein:

the core comprises a core material comprising an emulsifiable fragrance, and
the shell comprises in polymerized form a monomer blend comprising:

i) a monomer (I) selected from the group consisting of:

- a monoethylenically unsatured monomer,
- dimethyldiallyl ammonium chloride (DMDAAC), and
- mixtures thereof

and

ii) a monomer (II) which is a polyethylenically unsatured monomer,

wherein monomer (II) is such that the microcapsule provides for a fragrance leakage of less than about 70% when tested upon storage for 4 weeks at 40ºC according to the method disclosed in example 7, when the microcapsule is prepared according to the procedure disclosed in example 1 and the microcapsule encapsulates a fragrance material no. 2 as defined in example 1, and
wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.
In one aspect, the present invention relates to a microcapsule having an average particle size comprised between 7.5 and 50 microns, said capsule comprising a core and a polymeric shell enclosing said core, wherein:

the core comprises a core material comprising an emulsifiable fragrance, and
the shell comprises in polymerized form a monomer blend comprising:

i) a monomer (I) selected from the group consisting of:

- a monoethylenically unsatured monomer,
- dimethyldiallyl ammonium chloride (DMDAAC), and
- mixtures thereof

and

ii) a monomer (II) which comprises one or more methacrylic esters derived from polyhydric linear or branched $C_2$-$C_{24}$ alcohols or from linear or branched $C_2$-$C_{24}$ polyethylene glycols and which
A1. contains two or more methacrylate ester groups per monomer, and
B1. has a molecular weight which, once divided by the number of methacrylate ester groups, gives a value of more than 85 and lower than 135,

wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.
In one aspect, the present invention relates to a microcapsule as defined above, wherein said monoethylenically unsatured monomer (I) is selected from the group consisting of:

a) $C_3$-$C_6$ monoethylenically unsatured mono- or poly carboxylic acids,
b) amides of $C_3$-$C_6$ monoethylenically unsatured mono- or poly carboxylic acids;
c) optionally mono- or polysubstituted $C_1$-$C_{24}$ linear or branched alkyl esters of $C_3$-$C_6$ monoethylenically unsatured mono- or poly carboxylic acids,
and
d) optionally mono- or polysubstituted $C_3$-$C_6$ cycloalkyl esters of $C_3$-$C_6$ monoethylenically unsatured mono- or poly carboxylic acids,
wherein optional substituents are selected from the group consisting of:

- OH
- OR,
- C(O)R,
- $NH_2$,
- NHR,
- $NR_2$,
- $NR_3^+$,
- $C_5$-$C_6$ aromatic or heteroaromatic rings, and $C_3$-$C_{10}$ cyclo- or heterocyclic alkyl, wherein R is $C_1$-$C_4$ alkyl.

In one aspect, the present invention relates to a microcapsule as defined above, wherein said monoethylenically unsatured monomer (I) is selected from the group consisting of: methacrylic acid, methyl methacrylate, ethyl methacrylate, isopropyl methacrylate, isobornyl methacrylate and mixtures thereof, preferably from the group consisting of methacrylic acid, methyl methacrylate, ethyl methacrylate and mixtures thereof. In one aspect, the present invention relates to a

microcapsule as defined above, wherein said monoethylenically unsatured monomer (I) comprises a combination of methacrylic acid and methyl or ethyl methacrylate.

In one aspect, the present invention relates to a microcapsule as defined above, wherein said monomer (II) comprises one or more monomers selected from the group consisting of 1,4-butylene glycol dimethacrylate; 1,3-butylene glycol dimethacrylate; pentaerythritol trimethacrylate; glycerol trimethacrylate; 1,2-propylene glycol dimethacrylate, 1,3-propylene glycol dimethacrylate, ethylene glycol dimethacrylate, diethylene glycol dimethacrylate; glycerol dimethacrylate; trimethylolpropane trimethacrylate; ethoxylated pentaerythritol tetramethacrylate; divinylbenzene, and trivinylbenzene in an amount of at least 10% by weight over the combined weight of all monomers (II) present in the blend.

In one aspect, the present invention relates to a microcapsule as defined above, wherein said monomer (II) comprises at least 1,4-butylene glycol dimethacrylate or ethylene glycol dimethacrylate.

In one aspect, the present invention relates to a microcapsule as defined above, wherein said monomer (II) consists of 1,4-butylene glycol dimethacrylate or ethylene glycol dimethacrylate.

In one aspect, the present invention relates to a microcapsule as defined above, wherein said emulsifiable fragrance comprises a combination of at least four distinct emulsifiable fragrances.

In one aspect, the present invention relates to a microcapsule as defined above, wherein the core material further comprises a perfumery acceptable solvent.

In one aspect, the present invention relates to a microcapsule as defined above, wherein the core-to-shell weight ratio is comprised between 50:1 and 1:1.

In one aspect, the present invention relates to a microcapsule as defined above, wherein

- monomer (I) comprises, preferably consists of, a combination of methacrylic acid with methyl or ethyl methacrylate, and
- monomer (II) comprises, preferably consists of, 1,4-butane diol dimethacrylate or ethylene glycol dimethacrylate.

In one aspect, the present invention relates to a microcapsule as defined above, wherein

- monomer (I) comprises, preferably consists of, a combination of methacrylic acid with methyl or methacrylate, in an amount between 50% and 60% by weight, and
- monomer (II) comprises, preferably consists of, 1,4-butane diol dimethacrylate or ethylene glycol dimethacrylate in an amount between 40% and 50% by weight,
  wherein percentages are expressed over the combined weight of monomers (I) and (II) in the blend.

In one aspect, the present invention relates to a microcapsule as defined above, wherein

- monomer (I) comprises, preferably consists of, a combination of methacrylic acid between 35% and 45% by weight, and methyl or ethyl methacrylate between 11 % and 17% by weight, provided that the combination is in an amount between 50% and 60% by weight
  and
- monomer (II) comprises, preferably consists of, 1,4-butane diol dimethacrylate or ethylene glycol dimethacrylate in an amount between 40% and 50% by weight,
  wherein percentages are expressed over the combined weight of monomers (I) and (II) in the blend.

In one aspect, the present invention relates to a microcapsule as defined above, wherein

- monomer (I) consists of a combination of methacrylic acid 40% by weight, methyl or ethyl methacrylate 16% by weight, and
- monomer (II) consists of 1,4-butane diol dimethacrylate 44% by weight or ethylene glycol dimethacrylate 44% by weight,

wherein percentages are expressed over the combined weight of monomers (I) and (II) in the blend.

In one aspect, the shell comprises in polymerized form a monomer blend comprising, preferably consisting of methacrylic acid, methyl or ethyl methacrylate and 1,4-butane diol dimethacrylate or ethylene glycol dimethacrylate as defined above.

In one aspect, the present invention relates to a process for the manufacture of a microcapsule as defined above, which comprises the following steps:

a) preparing an oil-in-water emulsion having an oil phase and a water phase, said emulsion comprising:

- a polymerization initiator,
- a fragrance material comprising the emulsifiable fragrance,
- the monomer blend,
- an emulsifier, and optionally
- one or more further ingredients to be encapsulated

b) triggering polymerization within the emulsion obtained in step a),
c) letting the polymerization propagate thereby obtaining microcapsules.

In one aspect, the present invention relates to a water-based dispersion comprising a microcapsule as defined above.
In one aspect, the present invention relates to a product comprising a microcapsule as defined above, which is selected from the group consisting of:

- a non-edible consumer goods product
- a household cleaner or laundry product
- a personal care product
- a cosmetic product

In one aspect, the present invention relates to the use of a polyethylenically unsatured monomer and which comprises one or more monomers selected from the group consisting of divinylbenzene, trivinylbenzene, and a methacrylic ester derived from polyhydric linear or branched $C_2$-$C_{24}$ alcohols or from linear or branched $C_2$-$C_{24}$ polyethylene glycols, to reduce leakage from a microcapsule comprising a core and a polymeric shell enclosing said core, wherein the core comprises a core material comprising an emulsifiable fragrance.

## Detailed Description of the Invention

Below, all percentages quoted are weight percent unless otherwise stated.
Unless otherwise indicated:

- "an" or "the emulsifiable fragrance" means one or more emulsifiable fragrances;
- "a" or "the monomer (I)" means one or more monomers (I);
- "a" or "the monomer (II)" means one or more monomers (II);
- "a" or "the perfumery acceptable solvent" means one or more perfumery acceptable solvents;
- "a" or "the polymerization initiator" means one or more polymerization initiators;
- "an" or "the emulsifier" means one or more emulsifiers;
- "an" or "the benefit agent" means one or more benefit agents.

Unless otherwise indicated, "core material" refers to the encapsulated content of the microcapsule. In one embodiment it comprises, preferably consists of, the emulsifiable fragrance as presently defined and one or more further (and optional) ingredients to be encapsulated. In one embodiment, further optional ingredients to be encapsulated comprise, preferably consist of, a perfumery acceptable solvent as presently defined and/or a benefit agent as defined below. In one embodiment, the core of a microcapsule of the invention preferably consists of a core material as presently defined.
Unless otherwise indicated, "fragrance material" refers to the pre-emulsification and prepolymerization composition comprising, preferably consisting of, the emulsifiable fragrance as presently defined.
Unless otherwise indicated, the terms "fragrance" and "perfume" may be interchangeably used and refer to olfactively active material(s) typically but not necessarily providing a pleasant smell. Exemplary fragrance mixtures (also commonly referred to as "materials" in the field of perfumery) suitable for use in the present invention are described more fully in S. Arctander, Perfume Flavors and Chemicals 1969. Vols. I and II, Montclair, N.J and in Allured's Flavor and Fragrance Materials 2007 ISBN 978-1-93263326-9 published by Allured Publishing Corp. The term "fragrance" comprises both naturally occurring molecules as well as synthetic chemicals that are known for use as perfumes.
Unless otherwise indicated, the language "methacrylate ester group" used in the context of monomer (II) definition is a substituent having a molecular weight (MW) of 85 mass units and represented by the following formula:

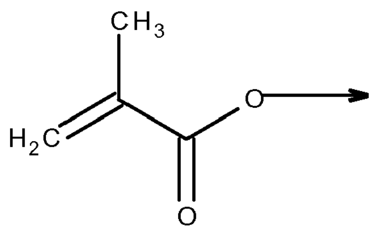

wherein the arrow indicates the point of attachment of the group to monomer (II).

Below, microcapsules that, like those of the present invention, have a core surrounded by, and enclosed within an external shell may also be referred to as "core shell microcapsules" or "core shell particles". This language is well known to those working in the field of encapsulated fragrances and perfumes and is structurally (and dimensionally) very different from other types of capsules such as conventional seamless soft capsules or two-piece hard capsules used e.g. in pharmacy to orally or rectally administrate substances to a subject. The microcapsules of the invention are also distinct from matrix particles obtainable e.g. by spray drying mixtures of water-soluble polymers and hydrophobic fragrances. In these particles in effect the hydrophobic material cannot be distinguished from the polymer composing the external surface of the particle but rather the two are admixed within the particle thereby forming a matrix.

Unless otherwise indicated, microcapsules of the invention are not intended for oral or rectal administration to human or animal subjects.

Unless otherwise indicated, all chemical terms have the meanings defined by the IUPAC Compendium of Chemical Terminology 2nd Edition Compiled by A D McNaught and A Wilkinson Blackwell Scientific Publications Oxford 1997 and IUPAC Nomenclature of Organic Chemistry, published by Blackwell Scientific Publications Oxford 1993 ISBN 0632034882.

The "microcapsule" of the invention has an average particle size equal to or greater than 7.5 microns (7.5μm), preferably equal to or greater than 10μm, preferably equal to or greater than 15μm, preferably equal to or greater than 20μm, preferably equal to or greater than 25μm. The "microcapsule" of the invention has an average particle size equal to or less than 50 microns (50μm), preferably equal to or less than 45μm, preferably equal to or less than 40μm. The "microcapsule" of the invention has an average particle size comprised between 7.5 microns (7.5μm) and 50 microns (50μm), or between 10μm and 50μm, or between 7.5μm and 45μm, or between 10μm and 45μm, or between 15μm and 45μm, or between 15μm and 40μm, or between 20μm and 45μm, or between 25μm and 45μm, or between 25μm and 40μm, or between 25μm and 35μm. The average particle size can be determined in several different ways, however the preferred technique is by light scattering using for example a Malvern Mastersizer or equivalent with the average particle size being taken as the median volume particle size D(v; 0.5) value.

The experimental results obtained by the present Applicant have shown that microcapsules whose shell comprises a monomer (I) as presently defined and having an average particle size lower than the values identified above display a worse (i.e. increased) leakage vis-a-vis microcapsules obtained using identical starting materials and process conditions but having an average particle size falling within the ranges disclosed in the present application. The reduced leakage of the microcapsules according to the present invention is particularly apparent when they are dispersed in liquid media such as in liquid laundry, personal care or cosmetic products.

Most microcapsules disclosed in the prior art have quite small average particle sizes. This is likely to be due to a technical belief that this aspect better copes with an efficient polymerization, thus leading to capsules with better properties. At the same time, it was also believed that average particle size did not have a significant impact on final capsule leakage. The experimental results obtained by the present Applicant have shown that no significant issues with polymerization step are met when encapsulating emulsion droplets of larger sizes and that running the manufacturing process so as to obtain larger average particle sizes brought about a significant advantage in terms of leakage.

The experimental results obtained by the present Applicant have also shown that it is recommendable not to work with microcapsules having an average particle size greater than the values identified above when wishing to obtain capsules endowed with better aesthetic appearance. In effect, it is believed to be preferable that the microcapsules be not visible at naked eye when deposited on a black surface. It has been found that microcapsules having an average particle size of greater than 50, e.g. equal to or greater than 60 or 70 microns can generate unpleasant aesthetic effects.

In one aspect, the present invention relates to a microcapsule comprising a core and a polymeric shell enclosing said core,
wherein the microcapsule is obtainable by polymerizing an emulsion of a fragrance material and a monomer blend,
wherein the fragrance material comprises the emulsifiable fragrance, and
wherein the shell monomer blend comprises:

i) a monomer (I) selected from the group consisting of:

- a monoethylenically unsatured monomer,
- dimethyldiallyl ammonium chloride (DMDAAC), and
- mixtures thereof

and

ii) a monomer (II) which is a polyethylenically unsatured monomer and which comprises one or more monomers selected from the group consisting of divinylbenzene, trivinylbenzene, and a methacrylic ester derived from polyhydric linear or branched $C_2$-$C_{24}$ alcohols or from linear or branched $C_2$-$C_{24}$ polyethylene glycols,
optionally wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.

In one embodiment, the microcapsule is obtainable by polymerizing an emulsion of a fragrance material, a monomer blend and further optional ingredients to be encapsulated. In one embodiment, said further optional ingredients to be encapsulated comprise one or more perfumery acceptable solvents as defined below and/or one or more benefit agents as defined below.

In one embodiment of the invention, the polymeric shell is formaldehyde-free. In one embodiment, formaldehyde-free means that free formaldehyde or formaldehyde-releasing ingredients are present in the shell in an amount of 0% by weight over the weight of the shell.

In one embodiment, the blend consists of a monomer (I) as presently defined and a monomer (II) as presently defined.

In one embodiment, the blend does not comprise any monoethylenically unsatured monomer other than a monomer (I) as presently defined.

In one embodiment, the blend does not comprise any polyethylenically unsatured monomer other than a monomer (II) as presently defined.

Unless otherwise indicated, the monoethylenically unsatured monomer (I) and the polyethylenically unsatured monomer (II) are polymerizable monoethylenically unsatured monomer (I) and polymerizable polyethylenically unsatured monomer (II), respectively.

In one embodiment, the monomer (I) comprises at least, preferably consists of a monoethylenically unsatured monomer or a mixture of monoethylenically unsatured monomers as presently defined.

In a different embodiment, the monomer (I) preferably comprises at least dimethyldiallyl ammonium chloride, more preferably in combination with a monoethylenically unsatured monomer or a mixture of monoethylenically unsatured monomers as presently defined.

In one embodiment, the monomer (I) is independently selected from the group consisting of: dimethyldiallyl ammonium chloride,
acrylic acid,
methacrylic acid,
maleic acid,
itaconic acid,
2-(diethylamino)ethyl methacrylate,
dimethylaminoethyl methacrylate,
2-(tert-Butylamino)ethyl methacrylate
N-[3-(dimethylamino)propyl]methacrylamide,
3-trimethylammonium propyl methacrylamide chloride,
methyl methacrylate
ethyl methacrylate,
n-propyl methacrylate,
isopropyl methacrylate,
tert-butyl methacrylate,
isobutyl methacrylate,
n-butyl methacrylate,
methacrylamide,
benzyl methacrylate
isobornyl methacrylate
cyclohexyl methacrylate
tetrahydrofuryl methacrylate,
glycidyl methacrylate
2-hydroxyethyl methacrylate

hydroxypropyl methacrylate
poly(ethylene glycol) methyl ether methacrylate
2-ethyl(2-oxoimidazolidin-1-yl)methacrylate
acryloxyethyltrimethyl ammonium chloride,
methacryloxyethyltrimethyl ammonium chloride and
mixtures thereof.

The household laundry personal care and cosmetic products into which microcapsules of the invention can be stored tend to have a range of pH values, from quite acidic pH values for limescale removing toilet cleaners, some hard surface cleaners and fabric conditioners, to quite alkaline pH for heavy duty laundry liquids. Thus in one particular aspect of the invention it may be advantageous that the monomers (I) are selected among methacrylates rather than acrylates since the former prove to be less susceptible to hydrolysis on prolonged exposure to acidic or alkaline pH and elevated storage temperatures than the latter. For example, the monoethylenically unsatured monomer (I) may be selected from the group consisting of those methacrylates that were found to present an advantageous balance of properties (such as cost, reactivity, hydrophilicity and mechanical properties). Thus monomer (I) comprises, preferably consists of, methacrylic acid, methyl methacrylate, ethyl methacrylate, isopropyl methacrylate, isobornyl methacrylate and mixtures thereof. For example it comprises, preferably consists of, a combination of methacrylic acid and methyl or ethyl methacrylate.

In one embodiment, monomer (II) is a polyethylenically unsatured monomer and it optionally comprises one or more monomers selected from the group consisting of divinylbenzene, trivinylbenzene, and a methacrylic ester derived from polyhydric linear or branched $C_2$-$C_{24}$ alcohols or from linear or branched $C_2$-$C_{24}$ polyethylene glycols. In the present disclosure, monomer (ii) may also be referred to as crosslinker due its function in the manufacturing of the capsule shell.

In one embodiment, the monomer (II) comprises at least, preferably consists of, a methacrylic ester derived from polyhydric linear or branched $C_2$-$C_{24}$ alcohols or from linear or branched $C_2$-$C_{24}$ polyethylene glycols.

In one embodiment, $C_2$-$C_{24}$ alcohols are preferably $C_2$-$C_{12}$ alcohols while $C_2$-$C_{24}$ polyethylene glycols are preferably $C_2$-$C_{12}$ polyethylene glycols.

In one embodiment, the methacrylic ester derived from polyhydric linear or branched $C_2$-$C_{24}$ alcohols or from linear or branched $C_2$-$C_{24}$ polyethylene glycols is selected from the group consisting of: 1,4-butylene glycol dimethacrylate; 1,3-butylene glycol dimethacrylate; pentaerythritol trimethacrylate; 1,2-propylene glycol dimethacrylate, 1,3-propylene glycol dimethacrylate, ethylene glycol dimethacrylate, diethylene glycol dimethacrylate; glycerol dimethacrylate, trimethylolpropane trimethacrylate, ethoxylated pentaerythritol tetramethacrylate, and 1,6-hexane diol dimethacrylate. For example, the group consists of 1,4-butylene glycol dimethacrylate; 1,3-propylene glycol dimethacrylate, ethylene glycol dimethacrylate and diethylene glycol dimethacrylate. For example, the group consists of 1,4-butylene glycol dimethacrylate and/or ethylene glycol dimethacrylate.

Accordingly, in one embodiment, the monomer (II) comprises at least, preferably consists of one or more monomers selected from the group consisting of 1,4-butylene glycol dimethacrylate; 1,3-butylene glycol dimethacrylate; pentaerythritol trimethacrylate; glycerol trimethacrylate; 1,2-propylene glycol dimethacrylate; 1,3-propylene glycol dimethacrylate; ethylene glycol dimethacrylate; diethylene glycol dimethacrylate; glycerol dimethacrylate; trimethylolpropane trimethacrylate; ethoxylated pentaerythritol tetramethacrylate; 1,6-hexane diol dimethacrylate; divinylbenzene; and trivinylbenzene. For example, one or more monomers selected from the group consisting of 1,4-butylene glycol dimethacrylate; 1,3-propylene glycol dimethacrylate, ethylene glycol dimethacrylate and diethylene glycol dimethacrylate. For example, one or more monomers selected from the group consisting of 1,4-butylene glycol dimethacrylate and/or ethylene glycol dimethacrylate.

For example, the monomer (II) comprises at least, preferably consists of 1,4-butylene glycol dimethacrylate. For example, the monomer (II) comprises at least, preferably consists of ethylene glycol dimethacrylate..

In a different embodiment, the monomer (II) comprises a methacrylic ester derived from polyhydric linear or branched $C_2$-$C_{24}$ alcohols or from linear or branched $C_2$-$C_{24}$ polyethylene glycols as defined above, preferably at least 1,4-butylene glycol dimethacrylate and/or ethylene glycol dimethacrylate, for example at least 1,4-butylene glycol dimethacrylate or ethylene glycol dimethacrylate, in an amount of at least 10%, preferably at least 20%, more preferably at least 30%, more preferably at least 40%, more preferably at least 50%, more preferably at least 60%, more preferably at least 70%, more preferably at least 80%, more preferably at least 90%, more preferably 100% by weight over the combined weight of all monomers (II) present in the blend.

In one embodiment, the monomer (II), preferably the blend, does not comprise:

- a polyethylenically unsatured monomer containing a carboxyl anhydride group (e.g. a monomer containing symmetric or asymmetric intermolecular anhydrides of monoethylenically unsatured monocarboxylic acids having 3 to 20 carbon atoms) and/or
- a polyethylenically unsatured monomer containing alkylenebis(meth)acrylamide group (e.g. N,N'-unsubstituted $C_{1-18}$ alkylene bis(meth)acrylamides or linear or cyclic N,N'-substituted $C_{1-18}$ alkylene bis(meth)acrylamides wherein substituents are selected from $C_{1-8}$ alkyl, $C_{1-8}$ hydroxyalkyl or polyoxy($C_{1-4}$)alkylene of 2 to 500 alkylene units or the

alkyl substituents together with the nitrogen atoms to which they are attached form a 5- to 8-membered ring).

In one embodiment, the monomer (II), preferably the blend, does not comprise 1,6-hexane diol dimethacrylate.

In one aspect, the present invention relates to a microcapsule having an average particle size comprised between 7.5 and 50 microns, said capsule comprising a core and a polymeric shell enclosing said core, wherein:

the core comprises a core material comprising an emulsifiable fragrance, and
the shell comprises in polymerized form a monomer blend comprising:

i) a monomer (I) selected from the group consisting of:

- a monoethylenically unsatured monomer,
- dimethyldiallyl ammonium chloride (DMDAAC), and
- mixtures thereof

and

ii) a monomer (II) which comprises one or more methacrylic esters derived from polyhydric linear or branched $C_2$-$C_{24}$ alcohols or from linear or branched $C_2$-$C_{24}$ polyethylene glycols and which
A1. contains two or more, for example 2 to 6, or 2 to 4 such as 2 or 3 or 4 methacrylate ester groups per monomer, and,
B1. has a MW (molecular weight, expressed as mass units) which, once divided by the number of methacrylate ester groups, gives a value of more than about 85, for example more than about 90, and equal to, or lower than about 135, such as lower than about 121,

wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.

As far as conditions A1 and B1 are met, all embodiments and definitions explicitly disclosed for monomer (II) in connection with other aspects of the present invention are to be understood to apply to this aspect too. However, in a particular embodiment of this aspect, monomer (II) may not be any one or more of 1,4-butylene glycol dimethacrylate; 1,3-butylene glycol dimethacrylate; pentaerythritol trimethacrylate; glycerol trimethacrylate; 1,2-propylene glycol dimethacrylate; 1,3-propylene glycol dimethacrylate; ethylene glycol dimethacrylate; diethylene glycol dimethacrylate; glycerol dimethacrylate; trimethylolpropane trimethacrylate and 1,6-hexane diol dimethacrylate.

In one aspect, the present invention relates to a microcapsule having an average particle size comprised between 7.5 and 50 microns, said capsule comprising a core and a polymeric shell enclosing said core, wherein:

the core comprises a core material comprising an emulsifiable fragrance, and
the shell comprises in polymerized form a monomer blend comprising:

i) a monomer (I) selected from the group consisting of:

- a monoethylenically unsatured monomer,
- dimethyldiallyl ammonium chloride (DMDAAC), and
- mixtures thereof

and

ii) a monomer (II) which is a polyethylenically unsatured monomer,

wherein monomer (II) is such that the microcapsule provides for a fragrance leakage of less than about 70%, such as less than about 60%, for example less than about 50%, when tested upon storage for 4 weeks at 40ºC according to the method disclosed in example 7, when the microcapsule is prepared according to the procedure disclosed in example 1 and the microcapsule encapsulates a fragrance material no. 2 as defined in example 1, and
wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.

Monomer (II) disclosed in the above aspect may be an example of a polyethylenically unsatured monomer which comprises one or more monomers selected from the group consisting of divinylbenzene, trivinylbenzene, and a methacrylic ester derived from polyhydric linear or branched $C_2$-$C_{24}$ alcohols or from linear or branched $C_2$-$C_{24}$ poly-

ethylene glycols.

Additionally, as far as the fragrance leakage condition is met, all embodiments and definitions explicitly disclosed for monomer (II) in connection with other aspects of the invention are to be understood to apply to this aspect too. However, in a particular embodiment of this aspect, monomer (II) may not be any one or more of 1,4-butylene glycol dimethacrylate; 1,3-butylene glycol dimethacrylate; pentaerythritol trimethacrylate; glycerol trimethacrylate; 1,2-propylene glycol dimethacrylate; 1,3-propylene glycol dimethacrylate; ethylene glycol dimethacrylate; diethylene glycol dimethacrylate; glycerol dimethacrylate; trimethylolpropane trimethacrylate; 1,6-hexane diol dimethacrylate; divinylbenzene; and trivinylbenzene.

In one embodiment, monomer (II) comprises an acrylic ester derived from polyhydric linear or branched $C_2$-$C_{24}$ alcohols or from linear or branched $C_2$-$C_{24}$ polyethylene glycols in an amount of less than 50%, preferably less than 40%, preferably less than 30%, preferably less than 20%, preferably less than 10%, more preferably less than 5%, more preferably less than 1% by weight over the combined weight of all monomers (II) present in the blend. Preferably, monomer (II), more preferably the blend, does not comprise any acrylic ester derived from polyhydric linear or branched $C_2$-$C_{24}$ alcohols or from linear or branched $C_2$-$C_{24}$ polyethylene glycols.

In one embodiment, the core preferably consists of a core material as presently defined.

In one embodiment, the emulsifiable fragrance comprises a combination of at least two, preferably at least four, more preferably at least eight distinct emulsifiable fragrances.

In one embodiment, the language "emulsifiable fragrance" means a fragrance which is endowed with hydrophobicity (measured for example in terms of its ClogP as defined in the present application) suitable for its partial or total emulsification in an oil-in-water emulsion. Typically, substances are emulsifiable according to the present invention when they are non-ionic and have a ClogP greater than 0 (limit not included), more preferably equal to, or greater than 2. To be endowed with an optimal volatility (which contributes to the fragrance's olfactive impact upon microcapsule rupture), it is preferable that emulsifiable substances have a ClogP equal to, or lower than for example 7, or equal to, or lower than for example 6, or equal to, or lower than for example 5, or equal to, or lower than for example 4.5. For example, preferred emulsifiable fragrances have a ClogP comprised between 2 and 5, for example in the range comprised between 2 and 4.5. Mixtures of two or more distinct fragrances, each having a different ClogP may also be emulsifiable. In this embodiment, all the fragrances in the mixture may present a ClogP within the ranges identified above. Alternatively, it is possible that some but not all the fragrances in the mixture may present a ClogP outside the ranges identified above provided that the final combination of fragrances presents a weight average ClogP within the ranges identified above.

In one embodiment, the language "the fragrance material comprises an emulsifiable fragrance" preferably means that more than 60% by weight of the fragrance material comprises one or more non-ionic fragrances, each having a ClogP octanol/water greater than 0.5. In another embodiment, the language "the fragrance material comprises an emulsifiable fragrance" preferably means more than 80%, preferably more than 90% by weight over the weight of the fragrance material comprises one or more non-ionic fragrances each having a ClogP (octanol/water) greater than 1, preferably greater than 1.5, more preferably greater than 2.0.

In one embodiment, the language "the core material comprises an emulsifiable fragrance" or "the fragrance material comprises an emulsifiable fragrance" means that the core material and the fragrance material do not comprise non-ionic fragrances having a ClogP greater than 6.

Water solubility of non-ionic organic molecules can be related to a theoretically derived parameter ClogP. ClogP refers to the octanol/water partitioning coefficient (P) of fragrance ingredients. The octanol/water partitioning coefficient of a fragrance is the ratio between its equilibrium concentrations in octanol and in water. The partitioning coefficients of fragrances are more conveniently given in the form of their logarithm to the base 10, logP. The logP values of many fragrances have been reported; for example, the Pomona92 database, available from Daylight Chemical Information Systems, Inc. (Daylight CIS), Irvine, Calif., contains many, along with citations to the original literature. The "calculated logP" (ClogP) is determined by the fragment approach of Hansch and Leo (cf., A. Leo, in Comprehensive Medicinal Chemistry, Vol. 4, C. Hansch, P. G. Sammens, J. B. Taylor and C. A. Ramsden, Eds., p. 295, Pergamon Press, 1990, incorporated herein by reference). The fragment approach is based on the chemical structure of each fragrance, and takes into account the numbers and types of atoms, the atom connectivity, and chemical bonding. The ClogP values, which are the most reliable and widely used estimates for this physicochemical property, are preferably used instead of the experimental logP values in the selection of fragrances which are useful in the present invention. There are several alternative methods of calculating or estimating $1_0$gP values which can show some variation in values. Even calculations within a given set of software may change over time as the algorithms are modified to give results which are closer to measured values. To remove any uncertainty the ClogP values reported herein are most conveniently calculated by the "CLOGP" program available within the Chemoffice Ultra Software version 9 available from CambridgeSoft Corporation, 100 CambridgePark Drive, Cambridge, MA 02140 USA or CambridgeSoft Corporation, 8 Signet Court, Swanns Road, Cambridge CB5 8LA UK. The ClogP values are preferably used instead of the experimental logP values in the selection of fragrances which are useful in the present invention. For natural oils or extracts the composition of such oils can be determined by analysis or using the compositions published in the ESO 2000 database published by BACIS (Boelens

Aroma Chemical Information Service, Groen van Prinsterlaan 21, 1272 GB Huizen, The Netherlands).

In one embodiment, the emulsifiable fragrance comprises fragrances each having a molecular weight greater than 100, preferably greater than 125 and lower than 325, preferably lower than 300, more preferably lower than 275. Fragrances having a molecular weight within these limits present a balanced volatility/hydrophobicity which makes them olfactively noticeable when the microcapsules release them but also sufficiently water-insoluble to be emulsified during encapsulation.

In the embodiment wherein the emulsifiable fragrance comprises more than one distinct emulsifiable fragrances (e.g. at least two or more), the combination of emulsifiable fragrances does not display a solid-liquid phase transition at a temperature comprised between -20°C and 120°C.

Unless otherwise indicated, emulsifiable fragrances resulting from combinations of the various definitions provided above are suitable for carrying out the present invention.

In one embodiment, the emulsifiable fragrance comprises at least 40%, preferably at least 60%, more preferably at least 80% by weight over the weight of the core.

In one embodiment, the fragrance material comprises at least 40%, preferably at least 60%, more preferably at least 80% by weight over the combined weight of the fragrance material itself and the other optional ingredients to be encapsulated, wherein such other ingredients are as defined above.

In one embodiment, the emulsifiable fragrance comprises bulky fragrance molecules. In one embodiment, more than 20%, preferably more than 40%, more preferably more than 60%, even more preferably more than 80% by weight over the weight of the fragrance material is comprised of one or more bulky fragrance molecules.

In one embodiment, bulky fragrance molecules are as disclosed in EP1894603A1, published on March 05, 2008 and having title "Encapsulation of bulky fragrance molecules".

In one embodiment, bulky fragrance molecules are emulsifiable fragrances as defined above. In one preferred embodiment, bulky fragrance molecules are emulsifiable fragrances at least as defined above with reference to fragrance ClogP. In one embodiment, bulky fragrance molecules have a molecular weight lower than 325.

In one embodiment, "bulky fragrance molecules" are selected from the group consisting of:

| Common name | CAS no. |
|---|---|
| amboryl acetate | 059056-62-1 |
| ambrox DL | 003738-00-9 |
| acetoketal | 005406-58-6 |
| ambrinol™ | 041199-19-3 |
| acetyl eugenol | 93-28-7 |
| acetyl vanillin | 881-68-5 |
| amber core™ | 139504-68-0 |
| ambretone™ | 37609-25-9 |
| ambrettolide™ | 28645-51-4 |
| anisyl acetate | 104-21-2 |
| bacdanol™ | 28219-61-6 |
| benzyl dimethyl carbinyl acetate | 151-05-3 |
| beta-homocyclocitral | 472-66-2 |
| boronal | 3155-71-3 |
| brahmanol™ | 72089-08-8 |
| benzophenone | 000119-61-9 |
| benzyl salicylate | 000118-58-1 |
| benzyl eugenol | 057371-42-3 |
| benzyl cinnamate | 000103-41-3 |
| borneol, L | 000464-45-9 |
| bornyl acetate | 000076-49-3 |
| bourgeonal | 18127-01-0 |
| calone™ | 28940-11-6 |
| cetalox™ | 003738-00-9 |
| celestolide™ | 013171-00-1 |
| camphene | 000079-92-5 |
| camphor gum powder synthetic | 000076-22-2 |
| cyclohexyl salicylate | 025485-88-5 |

(continued)

| Common name | CAS no. |
|---|---|
| cyclaprop™ | 017511-60-3 |
| cyclabute™ | 067634-20-2 |
| cyclacet™ | 005413-60-5 |
| coumarin | 000091-64-5 |
| cinnamyl cinnamate | 000122-69-0 |
| caryophyllene, beta | 000087-44-5 |
| caryophyllene | 000087-44-5 |
| caryophyllene acetate | 057082-24-3 |
| cedramber | 019870-74-7 |
| alpha cedrene | 469-61-4 |
| cedrenyl acetate | 1405-92-1 |
| cedryl acetate | 000077-54-3 |
| cedryl meth ether | 019870-74-7 |
| cedryl formate | 039900-38-4 |
| cineol 1,8 | 000470-82-6 |
| cineol, 1,4 | 000470-67-7 |
| cashmeran™ | 033704-61-9 |
| cedanol | 007070-15-7 |
| alpha copaene | 3856-25-5 |
| cyclohexyl anthranilate | 7779-16-0 |
| 2-cyclohexylidene-2-phenylacetonitrile | 10461-98-0 |
| cinnamyl phenyl acetate | 7492-65-1 |
| cedroxyde™ | 71735-79-0 |
| celery ketone | 3720-16-9 |
| civettone | 542-46-1 |
| clarycet | 131766-73-9 |
| coniferan | 67874-72-0 |
| delta-damascone | 57378-68-4 |
| damascol 4 | 4927-36-0 |
| delta-muscenone | 82356-51-2 |
| dihydrofloralol | 68480-15-9 |
| dihydrojasmone | 1128-08-1 |
| dynascone | 56973-85-4 |
| alpha-damascone | 24720-09-0 |
| gamma-damascone | 35087-49-1 |
| decahydro beta naphthyl acet, trans, | 010519-11-6 |
| doremox™ | 094201-73-7 |
| diphenyl oxide | 000101-84-8 |
| dibenzyl ketone | 000102-04-5 |
| dulcinyl™ | 055418-52-5 |
| ebanol™ | 67801-20-1 |
| exaltolide™ | 106-02-5 |
| exaltone™ | 502-72-7 |
| florasantol™ | 067739-11-1 |
| fenchyl alcohol | 001632-73-1 |
| florex | 069486-14-2 |
| fruitate™ | 080657-64-3 |
| fenchol | 22627-95-8 |
| fenchyl acetate | 13851-11-1 |
| floramat™ | 67801-64-3 |

(continued)

| Common name | CAS no. |
|---|---|
| fraistone™ | 6290-17-1 |
| galaxolide™ | 001222-05-5 |
| grisalva | 068611-23-4 |
| globalide™ | 34902-57-3 |
| green acetate | 88-41-5 |
| heliobouquet™ | 001205-17-0 |
| spirodecane™ | 6413-26-9 |
| hedione™ | 24851-98-7 |
| isocyclogeraniol | 68527-77-5 |
| iso cyclocitral | 1335-66-6 |
| iso borneol | 000124-76-5 |
| iso bornyl acetate | 000125-12-2 |
| isobornyl formate | 1200-67-5 |
| isobornyl methyl ether | 5331-32-8 |
| iso E super™ | 054464-57-2 |
| iso bornyl propionate | 002756-56-1 |
| iso proxen | 090530-04-4 |
| iso longifolanone | 014727-47-0 |
| iso butyl quinoline | 065442-31-1 |
| indolene | 068908-82-7 |
| gamma-ionone | 79-76-5 |
| alpha-ionone | 127-41-3 |
| dihydro iso jasmonate | 37172-53-5 |
| jasmelia™ | 58285-49-3 |
| karanal™ | 117933-89-8 |
| kephalis™ | 36306-87-3 |
| levosandol™ | 28219-61-6 |
| lilial™ | 80-54-6 |
| lyrame™ | 067634-12-2 |
| alpha iso methyl ionone | 1335-46-9 |
| methyl naphthyl ketone crystals | 000093-08-3 |
| methyl laitone | 94201-19-1 |
| methyl dioxolan | 06413-10-1 |
| methyl jasmonate | 1211-29-6 |
| muscone | 541-91-3 |
| musk ambrette | 83-66-9 |
| ethylene brassylate | 105-95-3 |
| musk thibetene | 145-39-1 |
| nerolin | 93-18-5 |
| naphthol iso butyl ether, beta | 002173-57-1 |
| nootkatone 98% | 004674-50-4 |
| neoproxen | 122795-41-9 |
| (12R,9Z)- nirvanolide™ | 22103-61-8 |
| nopol | 128-50-7 |
| nopyl acetate | 35836-72-7 |
| okoumal™ | 131812-67-4 |
| orriniff™ | 125352-06-9 |
| orivone™ | 16587-71-6 |
| palisandin | 2986-54-1 |
| pinene, alpha | 000080-56-8 |

(continued)

| Common name | CAS no. |
|---|---|
| pinene, beta | 000127-91-3 |
| phenyl ethyl phenyl acetate | 000102-20-5 |
| phantolid™ | 015323-35-0 |
| plicatone™ | 041724-19-0 |
| patchone | 98-52-2 |
| patchouly ketone | 98-53-3 |
| piperonyl acetate | 326-61-4 |
| polysantol™ | 107898-54-4 |
| precyclemone B | 52474-60-9 |
| romascone™ | 81752-87-6 |
| rhubofix™ | 041816-03-9 |
| sandalmysore core™ | 28219-60-5 |
| sandalore™ | 65113-99-7 |
| santalex T™ | 068877-29-2 |
| scentenal™ | 086803-90-9 |
| spirambrene™ | 12151-67-0 and 12151-68-1 |
| tonalid™ | 021145-77-7 |
| traseolide™ | 068140-48-7 |
| thymoxane | 707-29-9 |
| timberol™ | 70788-30-6 |
| trimofix O™ | 28371-99-5 |
| vanillin propylene glycol acetal | 068527-74-2 |
| vigoflor™ | 068480-11-5 |
| verdol™ | 13491-79-7 |
| veloutone | 65443-14-3 |
| veratraldehyde | 120-14-9 |
| veratricacid | 93-07-2 |
| vertenex™ | 32210-23-4 |
| violiff ™ | 87731-18-8 |
| yara | 000093-04-9. |

In one embodiment, optional ingredients to be encapsulated comprise one or more perfumery acceptable solvents. Solvents are conventionally used in the fragrance industry to dilute olfactively powerful ingredients and to facilitate the handling of solid ingredients by dissolving them and handling them as liquids, or simply as a diluent to reduce overall fragrance cost per unit weight. It is preferable to avoid diluting fragrance compositions intended for encapsulation if possible. Certain fragrances as presently defined that are liquid at room temperature (e.g. 20°C) are also endowed with solvent properties so that they can be used to dissolve benefit agents or other fragrance material ingredients (e.g. fragrances that are solid at room temperature).

Examples of perfumery acceptable solvents are benzyl benzoate, isopropyl myristate, dialkyl adipates, citrate esters (such as acetyl triethyl citrate, acetyl tributyl citrate and triethyl citrate), diethyl phthalate, propylene glycol dipropylene glycol, and butylene glycols.

In one embodiment, the one or more perfumery acceptable solvents comprise water-immiscible solvents, wherein water-immiscible preferably means a solubility in water of less than 10g/L.

In one embodiment, optional ingredients to be encapsulated comprise one or more benefit agents. Accordingly, in one embodiment, the core material further comprises one or more benefit agents. Certain fragrances as presently defined may also be endowed with additional properties that for certain applications may make their use desirable also as benefit agents.

Benefit agents are typically emulsifiable materials which can survive storage to deliver a benefit when used in household, personal care or cosmetic products.

Benefit agents may include natural extracts or materials which have therapeutic effects e.g. as relaxants or stimulants, such as natural oils or plant extracts which are beneficial to skin such as jojoba oil or almond oil are benefit agents. Materials which suppress or reduce malodour and its perception by odor absorption such as zinc ricinoleate

(CAS 13040-19-2) may be benefit agents. Materials which when added to the emulsion improve the properties of the core emulsion before encapsulation, or the properties of the capsules themselves such as organic density modifying agents such as triethyl citrate, sucrose octa-acetate or sucrose hexabutyrate di-acetate which can help to stabilize the capsules in liquid products. Benefit agents can include materials which act can as gelling agents for the core such as hydroxy fatty acids or the Sylvadear™ range of materials available from Arizona Chemicals. Materials which provide a warming or cooling effect such as described in Cosmetics and Toiletries 2005 Vol. 120 No 5 p105 by M Erman may also be benefit agents. Examples of such agents include but are not limited to: cyclohexane carboxamide N-ethyl-5-methyl-2-(1-methylethyl) known as WS3TM (CAS N° 39711-79-0); N 2,3-trimethyl-2-isopropylbutamide known as WS23TM (CAS 51115-67-4); menthyl lactate (CAS N° 59259-38-0); and (-)-menthoxypropane 1,2-diol known as cooling agent 10TM. Materials such as skin whitening agents may also be benefit agents according to the invention. Materials which act as insect repellents exemplified by ethylbutylacetylaminopropionate known as Merck's IR3535TM (CAS N° 52304-36-6); or N,N-diethyl toluamide (CAS N° 134-62-3); or 1-piperidirecarboxylic acid; 2-(2-hydroxyethyl)-1-methylpropyl ester known as Bayrepel™ (CAS N° 119515-38-7); or p-menthane-3,8-diol (CAS No 42822-86-6) or natural plant oils such as Tea Tree oil, neem oil, citronella oil, or eucalyptus oil are benefit agents. Materials which act as antimicrobial agents as exemplified by triclosan™ (CAS N° 3380-34-5), the methyl-ethyl, propyl and butyl para hydroxy benzoate esters (CAS N° 4247-02-3, 94-26-8, 94-13-3, 120-47-8, 99-76-3). Materials which act as UV absorbers such as octyl methoxycinnamate, Benzophenone 3, butylmethoxydibenzoylmethane, or bis ethylhexyloxyphenolmethoxyphenyltriazine may also be benefit agents. The materials listed above are intended to exemplify the benefit agents but are not intended to limit the benefit agents to this list. Mixtures of the above may also be considered as benefit agents of the invention. Thus it may be advantageous to combine UV absorbers with an insect repellent in a leave on personal care product or to combine an anti-fungal agent with a bactericide for broader antimicrobial protection. Moreover it is recognized that some materials may exhibit more than one benefit. Thus triethyl citrate may function as a solvent and as a density modifying agent.

In one embodiment, the one or more benefit agents are each a non-ionic substance having a ClogP greater than (and not including) 0, preferably greater than 0.5, more preferably greater than 2.

In one embodiment, the one or more benefit agents are each independently selected from the group consisting of:

- relaxants or stimulants, such as jojoba oil or almond oil,
- agents which suppress or reduce malodour and its perception by adsorbing odour such as zinc ricinoleate,
- agents improving microcapsule physical-chemical properties such as triethyl citrate, sucrose octa-acetate or sucrose hexabutyrate di-acetate,
- gelling agents such as hydroxy fatty acids or the Sylvaclear™ range of materials available from Arizona Chemicals,
- agents which provide a warming or cooling effect such as cyclohexane carboxamide N-ethyl-5-methyl-2-(1-methylethyl); N 2,3-trimethyl-2-isopropylbutamide; menthyl lactate; (-)-menthoxypropane 1,2-diol,
- insect repellents such as ethylbutylacetylaminopropionate; N,N-diethyl toluamide; 1-piperidinecarboxylic acid; 2-(2-hydroxyethyl)-1-methylpropyl ester; p-menthane-3,8-diol, Tea Tree oil, neem oil, citronella oil, and eucalyptus oil,
- antimicrobial agents such as triclosan™ compound having CAS N° 3380-34-5, or the methyl, -ethyl, propyl and butyl para hydroxy benzoate esters,
- UV absorbers such as octyl methoxycinnamate, butylmethoxydibenzoylmethane, and bis ethylhexyloxyphenolmethoxyphenyltriazine.

Thickness of the shell may play an important role in capsule permeability and performance. This parameter is particularly important for friable capsules which release fragrances by breaking. If the shell is too thick the capsules will not break in use, however if the shell is too thin the capsules will not survive the manufacturing and shipping involved in making a product. In one embodiment, the present invention relates to a microcapsule wherein the core-to-shell weight ratio is comprised between 50:1 and 1:1, preferably between 30:1 and 1:1, even preferably between 20:1 and 1:1, more preferably between 10:1 and 1:1.

In one embodiment, the present invention relates to a microcapsule wherein the microcapsule is substantially spherical.

The microcapsules of the invention may be prepared using a range of conventional methods known to those skilled in the art for making microcapsules, such as coacervation, interfacial polymerization, free radical polymerization, or polycondensation. These techniques are well-know, see e.g., US3516941, US4520142, US4528226, US4681806, US4145184; GB-A-2073132; WO99/17871; and MICROENCAPSULATION Methods and Industrial Applications Edited by Benita and Simon (Marcel Dekker, Inc. 1996).

In one embodiment, the microcapsules of the invention can conveniently be prepared through a polymerization step. In one embodiment, the polymerization is conventional radical polymerization, living radical polymerization or telomerization. Such radical polymerization processes are known to persons skilled in the art and are further described in Moad, Graeme; Solomon, David H.; The Chemistry of Radical Polymerization, 2nd ed.; Elsevier, 2006 which is

incorporated herein by reference.

The main difference of a living radical polymerization, as compared to conventional radical polymerization, is the introduction of a reversible radical termination process. By relying on equilibrium between dormant (reversibly terminated) and active (radical-containing) chains, the instantaneous concentration of propagating radicals is low, such that the rates of irreversible termination and degradative chain transfer are significantly reduced. While some degree of termination is unavoidable, the presence of primarily dormant chains at the completion of polymerization allows the preparation of polymers with controlled molecular weight, composition, and chain topology. Examples of living radical polymerization processes include but are not restricted to atom transfer radical polymerization, nitroxide-mediated radical polymerization, reversible addition-fragmentation chain transfer polymerization and other related processes involving a degenerative transfer, such as macromolecular design via interchange of xanthates. Addition of specific compounds and their relative amounts to obtain these living radical polymerization processes are well-known to a person skilled in polymerization. Further description can be found in the literature for example in Braunecker, Wade A.; Matyjaszewski, Krzysztof; "Controlled/Living Radical Polymerization: Features, Developments, and Perspectives"; Progress in Polymer Science 2007, Volume 32, Issue 1, Pages 93-146.

Telomerization is a radical polymerization reaction where a chain transfer limits the size of the formed polymer, which is thus an oligomer named telomer. Telomerization requires an active chain transfer agent named a telogen or a regulator which is used in typical amounts from 0.05 to 0.5 % based on the weight of monomers to be polymerized. Examples of telogens include but are not restricted to mercapto compounds like 2-ethylhexyl thioglycolate, tert-dodecyl mercaptan, thioglycolic acid and 2-mercaptoethanol. Telomerization is described further in the literature for example in Boutevin, Bernard; "From Telomerization to Living Radical Polymerization"; Journal of Polymer Science Part A: Polymer Chemistry 2000, Volume 38, Issue 18, Pages 3235-3243 which is incorporated herein by reference.

According to the process of the invention an oil-in-water emulsion may be prepared by mixing and dissolving the oil soluble ingredients into a homogeneous solution while separately mixing and dissolving the water soluble ingredients into a homogenous solution. The emulsion may be finally obtained by mixing with a high shear mixer for sufficient time to create a stable emulsion of the correct particle size. At the same time the emulsion may be purged with nitrogen or other inert gas. Once the air has been removed the temperature is elevated to initiate the polymerization. The exact temperature and rate of temperature increase is determined by the initiator or combination of initiators to be used. Typically polymerization temperatures are between 40°C to 90°C. The rate of polymerization can be contrdled in a known manner by appropriate choice of the temperature and amount of polymerization initiator for the particular monomers and initiator in an experiment. Once the polymerization temperature has been reached, polymerization continues for a further period, for example 2 to 6 hours, in order to complete the reaction of the monomers.

There are many variations on this basic encapsulation procedure. For example emulsification may be achieved using a variety of methods all well known to those skilled in the art. For example low shear mixing combined with the addition of surfactants can form an emulsion alternatively initial high shear mixing might be used to create the desired particle size followed by low shear agitation with a protective colloid to keep the emulsion dispersed. Additional initiator can be added later in the polymerization to reduce the level of residual monomer. Monomers may be added during the course of the reaction to control dosage. Salts may be added e.g. to buffer the pH.

In one embodiment, polymerization is triggered by inducing decomposition of radical initiators (e.g. thermal decomposition), redox initiators, photoinitiators or combinations of these. Polymerization may be initiated either in the oil phase or the water phase of the emulsion depending on the choice of the initiator(s). It is also possible to initiate polymerization in the two phases separately by appropriate choice of initiator and conditions.

In one embodiment, step b) comprises:

- subjecting the oil-in-water emulsion to heat and/or UV light and/or
- triggering a redox reaction within the oil-in-water emulsion.

In one embodiment, the emulsifier is selected from the group consisting of:

- one or more surfactants, and/or
- one or more protective colloids.

Protective colloids and/or surfactants are conventionally used in emulsion polymerization and in suspension polymerization to stabilise oil-in-water emulsions created by mechanical agitation while the polymerization occurs.

In one embodiment, the emulsion may comprise further optional ingredients that are to be encapsulated. In one embodiment, such optional ingredients comprise, preferably consist of, one or more water-immiscible perfumery acceptable solvents as presently defined above and/or one or more benefit agents as presently defined.

In one embodiment, the emulsifier is a surfactant. Surfactants are amphiphilic molecules i.e. they consist of a hydrophobic part and a hydrophilic part. The hydrophobic part is generally a hydrocarbon alkyl chain of between 8 to

20 carbon atoms which may be linear or branched and may contain aromatic rings. The hydrophilic part of the molecule can be a non-ionic, anionic cationic or zwitterionic group. Commonly used nonionic hydrophilic groups include polyethoxylated and polypropoxylated groups of different chain lengths typically 3-50 ethylene units long or mixtures of the two, or glycerides or saccharides as either alkyl esters or alkyl ethers. Examples of non-ionic emulsifiers include the Neodol® polyethoxylated alcohols from Shell or the Cremophor® polyethoxylates from BASF or the Plantacare® range of alkyl polyglycosides from Cognis or the sugar esters from Mitsubishi Kagaku Corporation. Anionic hydrophilic parts generally consist of ammonium or alkali metal salts of sulphate, sulphonate, sulphosuccinate, phosphate or carboxylic acid groups. Examples of such surfactants include sodium alkyl benzene sulphonate, sodium alkyl sulphates, dialkyl sulphosuccinates or sodium carboxylates. Cationic surfactants are usually quaternary ammonium salts of halide or methosulphate anions such as monoalkyly trimethyl ammonium chlorides available commercially under the name Prapagen@ from Hoescht salts. The selection of the appropriate surfactant or mixture of surfactants to achieve the appropriate particle size emulsion is well known to those skilled in the art and is described in "Emulsion Science and Technology by T F Tadros et al Wiley-VCH 2009 ISBN 3527325255. A detailed review of surfactants suitable in the process of the invention can also be found in WO2010119020 (already quoted in the background section), from page 9, line 6 till page 14, line 10. In particular, reference can be made to the alcohol alkoxylates or alcohol phenol alkoxylates of formula (V) and the specific examples thereof disclosed in WO2010119020 page 12, line 19 till page 13, line 13.

In one embodiment, the emulsifier is a protective colloid.

In one embodiment, the protective colloid has an average molecular weight comprised between 500 and 1.000.000 g/mol, preferably between 1.000 and 500.000 g/mol.

In one embodiment, the protective colloid is independently selected from the group consisting of:

- cellulose derivatives such as hydroxyethylcellulose, carboxymethylcellulose and methylcellulose,
- polyvinylpyrrolidone,
- copolymers of N-vinylpyrrolidone,
- polyvinyl alcohols obtainable by full to partial hydrolyses of polyvinyl acetates,
- polyacrylic and/or polymethacrylic acid,
- copolymers of acrylic acid and methacrylic acid,
- ionic colloids such as sulphonic-acid-group-containing water-soluble polymers (e.g. 2-acrylamido-2-alkylsulphonic acids and styrene sulphonic acids), and
- mixtures thereof.

In one embodiment, the one or more protective colloid comprises polyvinyl alcohols obtainable by full to partial hydrolyses of polyvinyl acetates.

In one embodiment, the one or more protective colloid is present in an amount comprised between 0.1% and 10% by weight over the weight of the water phase of the oil-in-water emulsion.

In one embodiment, each one of the protective colloid is a water-soluble protective colloid. Preferably, this means that the colloid has solubility in water of at least 5 g/L.

Polymerization generally occurs in the presence of polymerization initiators which form radicals. It is known that radicals can be generated by thermal decomposition of compounds such as peroxy and azo compounds, or by photolysis with UV radiation or by redox reactions. Initiators suitable for performing the present invention can be soluble in the oil phase and/or the aqueous phase of the emulsion.

In one embodiment, the one or more polymerization initiators are:

- one or more thermal polymerization initiators, and/or
- one or more photopolymerization initiators, and/or
- one or more redox initiators, wherein each redox initiator comprises a radical-generating reductant/oxidant pair.

In one embodiment, the one or more polymerization initiators comprise one or more thermal polymerization initiators in an amount comprised between 0.1% and 5% by weight over the combined weight of monomers (I) and (II) in the blend.

In one embodiment, the thermal polymerization initiator is selected from the group consisting of: dilauroyl peroxide,
benzoyl peroxide,
α ,α'-azoisobutyronitrile,
2,2'-azobis(2.4-dimethyl valeronitrile),
dimethyl 2,2'-azobis(2-methylpropionate),
1,1'-azo-bis-1-Cyclohexanenitrile,
di-tert-butyl peroxide (CAS: 75-91-2),

potassium persulphate,
ammonium persulfate,
4,4'-azobis(4-cyanovaleric acid),
2,2'-azobis[2-(2-imidazolin-2-yl)propane]dihydrochloride,
2,2'-azobis(2-methylpropionamidine)dihydrochloride,
2,2'-azobis[2-(2-imidazolin-2-yl)propane],
**[0118]** 2,2'-azobis[2-methyl-N-(2-hydroxyethyl)propionamide], and
mixtures thereof.

In one embodiment, the polymerization initiator comprises one or more photopolymerization initiators in an amount comprised between 0.5% and 5% by weight over the combined weight of monomers (I) and (II).

In one embodiment, the photopolymerization initiator is selected from the group consisting of:

alpha hydroxyl ketones,
alpha amino ketones,
alpha and beta naphthyl carbonyl compounds,
benzoin ethers such as benzoin methyl ethers,
benzophenone,
acetophenone,
benzaldehyde,
xanthone,
9,10-anthraquinone,
1-hydroxy-cyclohexyl-phenyl-ketone (Irgacure™ 184), and
mixtures thereof.

In one embodiment, the polymerization initiator comprises one or more redox initiators wherein in the radical-generating reductant/oxidant pair

- the oxidant is present in an amount comprised between 0.01% and 3.0%, preferably between 0.02% ando 1.0% and more preferably between 0.05% and 0.5% by weight over the combined weight of monomers (I) and (II) in the blend, and/or
- the reductant is present in an amount comprised between 0.01% and 3.0%, preferably between 0.01% and 0.5% and more preferably between 0.025% and 0.25% by weight over the combined weight of monomers (I) and (II) in the blend.

In one embodiment, the redox initiator comprises one or more oxidant selected from the group consisting of:

- salts of peroxodisulfuric acid such as sodium monopersulfate, sodium persulfate, potassium persulphate, ammonium persulfate,
- Cumene hydroperoxide,
- tert-butyl hydroperoxide,
- di-tert-amyl peroxide,
- tert-butyl peroxybenzoate,
- t-amyl hydroperoxide, and
- hydrogen peroxide.

In one embodiment the redox initiator comprises one or more reductants selected from the group consisting of:

- sodium sulphite,
- sodium metabisulphite,
- sodium formaldehyde sulphoxylate,
- ascorbic acid, and
- sodium dithionite.

The microcapsules of the invention may also comprise on their surface (e.g. surface grafted) deposition aids, i.e. aids aiming to optimize the deposition of microcapsule on the intended substrate. Examples and use of deposition aids on microcapsules are for example disclosed in EP21558474, EP1572767, EP2188364 and EP1019478.

In one embodiment, the deposition aid comprises a polymeric deposition aid. Examples may be synthetic or natural polymers or combinations thereof (e.g. through partial chemical modification of natural polymers).

In one embodiment, the deposition aid comprises a peptide, a protein, or a chemical derivative thereof, providing for a binding to the intended substrates. For example cellulases bind to cotton while proteases bind to wool, silk or hair.

In one embodiment, the deposition aid comprises a polysaccharide or a chemical derivative thereof. The polysaccharide preferably has a [beta]-1,4-linked backbone. Preferably the polysaccharide is selected from the group consisting of a cellulose, a cellulose derivative, or another [beta]-1 ,4-linked polysaccharide binding to cellulose, such as polymannan, polyglucan, polyglucomannan, polyxyloglucan and polygalactomannan or mixtures thereof. More preferably, the polysaccharide is selected from the group consisting of polyxyloglucan and polygalactomannan. Highly preferred polysaccharides are selected from locust bean gum, tamarind gum, xyloglucan, non-ionic guar gum, cationic starch and mixtures thereof. Most preferably, the deposition aid is locust bean gum, or chemical derivatives thereof.

Deposition aids can be bound to a pre-formed microcapsule of the invention or they can be part of the materials used in the polymerization manufacturing process. The deposition aid may be physically and/or chemically bonded to the microcapsules. For example the deposition aid may be attached to the particle by means of a covalent bond, entanglement or adsorption, preferably by a covalent bond or entanglement and most preferably by means of a covalent bond.

By entanglement as used herein is meant that the deposition aid is partially buried within the interior of the microcapsule. This is obtained by adding the deposition aid to the emulsion e.g. before the polymerization is triggered. By letting the polymerization propagate, part of the deposition aid remains entrapped and bound in the extending polymer that will form the microcapsule shell whilst the remainder is free to extend into the aqueous phase of the emulsion. In this manner, the deposition ai dis only partially exposed at the microcapsule surface.

By adsorption as used herein is meant adsorption (i.e. physical binding) of the deposition aid to the already-formed microcapsule by means of, for example, hydrogen bonding, Van Der Waals or electrostatic attraction between the deposition aid and the microcapsule. The deposition aid is thus external to the microparticle and is not, to any significant extent, within the shell and/or within the microcapsule core.

Preferably, the deposition aid is present in an amount comprised between 0.1% and 10% by weight over the dry weight of a microcapsule.

In one aspect, the present invention relates to a product comprising a microcapsule as defined above which is selected from the group consisting of a non-edible consumer goods product, a household cleaner or laundry product, a personal care product and a cosmetic product.

Unless otherwise indicated, non-edible means not tit to be ingested by humans or animals. This includes non-food products that may accidentally be swallowed during normal use. Notably, included within the definition of non-edible products are products for dental and oral care, such as toothpastes, mouth washes and lip balms which although not intended for ingestion may nevertheless accidentally enter the gastro-intestinal tract.

The formulations and ingredients of liquid household, laundry, personal care and cosmetic products in which microcapsules of the invention may be used are well known to those skilled in the art, reference may be made to the following works:

- Formulating Detergents and Personal Care Products A guide to Product Development by L Ho Tan Tai, ISBN 1-893997-10-3 published by the AOCS Press
- Volume 67 of the Surfactant Science Series Liquid Detergents ISBN 0-8247-9391-9 (Marcel Dekker Inc),
- Harry's Cosmeticology published by CHS Press 8th Edn. 2000 ISBN 0820603724.

In one embodiment, the present invention relates to a product comprising a microcapsule as presently defined, which is a personal care or cosmetic product. The product can be applied to the skin, hair and nails either as leave on or rinse off product. Personal care and cosmetic compositions include powders, creams, emulsions, lotions, gels and oils for the skin (face, hands, feet etc), tinted bases (liquids and pastes) and liquid impregnated tissues; products for applying and removing make-up from the face and eyes; hair care products including: hair tints and bleaches, products for waving, straightening, setting and fixing hair; shaving products including: creams, foams mousses and depilatory products; sun bathing products and products for tanning without the sun; deodorant and antiperspirant products.

In one preferred embodiment a personal care or cosmetic product is selected from the group consisting of a shaving aid, a shampoo, a hair-conditioner product, a leave-on-skin-care product, a skin cleansing or washing product (such as a rinse-off skin cleansing or washing product), a moist tissue and a body spray, deodorant or antiperspirant.

Shaving aids specifically include foams, gels, creams and bars (reference can be made for example to US7,069,658, US6,944,952, US6,594,904, US6,182,365, US6,185,822, US6,298,558 and US5,113,585).

Shampoos and hair conditioners specifically include two-in-one shampoos and shampoos especially formulated for dry or greasy hair or containing additives such as antidandruff agents. Hair conditioners may be rinse off or leave on hair conditioners also included are hair tonics, bleaches colorants, setting and styling products. Reference can be made for example to US 6,162,423, US 5,968,286, US 5,935,561, US 5,932,203, US 5,837,661, US 5,776,443, US 5,756,436, US 5,661,118, US 5,618,523.

Leave-on-skin-care products comprise skin washing products, moist tissues, body sprays, deodorants and antiperspirants.

Skin washing products specifically include beauty and hygiene bar soaps, shower gels, liquid soaps, body washes, exfoliating gels and pastes (reference can be made for example to US3,697,644; US4,065,398; US4,387,040).

Moist tissues (wipes) specifically include skin cleansing wipes, baby wipes, make-up removal wipes and skin refreshing wipes (reference can be made for example to US4,775,582; WO02/07701; WO2007/069214 and WO95/16474).

Body sprays, deodorants and antiperspirants specifically include sticks, liquid roll-on applicators and pressurized sprays.

In one embodiment, the present invention relates to a product comprising a microcapsule as presently defined, which is a household cleaner or laundry product. Examples of household cleaners and laundry products which may comprise microcapsules of the invention include:

- hard surface cleaners such as cleaners for floors, solid work surfaces, tiled surfaces, crockery by hand or machine washing and mirrors and glass,
- soft furnishing treatments such as liquid cleaners and refresher products such as odour treatment agents as exemplified by Febreze® (P&G),
- powdered laundry detergents, detergent tablets and bars, laundry detergent liquids include light duty liquids, heavy duty liquids, concentrated liquid detergents, non or low aqueous laundry liquids and more specialised cleaners for woollen or dark garments,
- fabric softeners and pre- and post-wash treatments such as tumble drier sheets, ironing waters and wash additives.

Preferably, a household cleaner or laundry product is selected from the group consisting of a fabric softener, a fabric conditioner and a laundry detergent.

Household cleaners may be in the form of cream cleaners, isotropic liquid cleaners, spray cleaners and pre-moistened surface cleaning wipes (reference can be made for example to WO91/08283, EP743280, WO96/34938, WO01/2351 and WO99/28428).

Fabric softeners and conditioners specifically include both conventional diluted (e.g. 2% to 8% by weight) liquid active concentration softeners and concentrated (e.g. 10% to 40% by weight) liquid active concentration softeners as well as fabric conditioners which may contain ingredients to protect colors or garment shape and appearance (reference can be made for example to US 6,335,315, US 5,674,832, US 5,759,990, US 5,877,145, US 5,574,179).

Laundry detergents, particularly liquid laundry detergents, specifically include light duty liquid detergents and heavy duty liquid detergents which may be structured multi-phase liquids or isotropic liquids and which may be aqueous or non-aqueous liquids. These liquids may be in bottles or unit dose sachets and they may optionally contain bleaching agents or enzymes (reference can be made for example to US 5,929,022, US 5,916,862, US 5,731,278, US 5,470,507, US 5,466,802, US 5,460,752, and US 5,458,810).

The products of the invention may contain water and/or surface active material, either as an emulsifier, if the product is an emulsion, or as a detergent active material if the product has some kind of cleaning function. In certain embodiments the concentration of surface active material in the product will be within the range 0.1-60% by weight; usually the level of surface active material will be 50% by weight or lower; for most products the level of surface active material will be 30% by weight or lower. On the other hand, the level of surface active material will usually be at least 0.1 % by weight preferably greater than 1.0% and more preferably greater than 3.0% by weight. Certain product formulations are water sensitive (e.g. anti-perspirant, deodorant formulations, non-aqueous liquids packaged in water soluble polyvinyl alcohol films), and for these applications it may be desirable to spray dry the microcapsules to remove water, before the microcapsules are incorporated in the product formulation. For products which have a cleaning function it is likely the level of surface active material will be higher, typically greater than 10% by weight and preferably greater than 15% by weight. All percentages are expressed by weight over the weight of the product.

Examples of leave-on products containing emulsifiers are: hand and body lotions, make up removing lotions, skin creams, sunscreen products and sunless tanning products and domestic freshener sprays. Also included are articles of manufacture impregnated with liquids, for example pads or wipes impregnated with lotions for make-up application or removal, or to apply sunscreen compounds or sunless tanning agents, for personal cleansing e.g. as moist toilet tissue or baby wipes.

Examples of personal cleansing products containing detergents are: shampoos, body washes, liquid soaps. Some cleaning products may be considered leave on products even though they are used for cleansing if there is no rinsing or further cleaning action after use. Baby wipes are an example, although used for cleaning the liquid deposited on the skin is not removed by rinsing.

The non-rinsed cosmetic, toiletry and personal care compositions described herein can contain various emulsifiers which are useful for emulsifying the various components of the products. Suitable emulsifiers can include any of

a wide variety of non-ionic, cationic, anionic, and zwitterionic surface active materials as disclosed in publications such as McCutcheon's, Detergents and Emulsifiers, North American Edition (1986), published by Allured Publishing Corporation and in the following patents: U.S. 5,011,681; U.S. 4,421,769; and U.S. 3,755,560.

Experimental evidence shows that the composition of certain products such as setting lotions, eau de toilettes, body spray aerosols, hair foams, which contain short hydrocarbon chain alcohols may negate the benefit brought about by the microcapsules presently disclosed. Therefore, it is preferable that the products containing the present microcapsules do not also contain significant amounts (e.g. more than 2.5% or more than 5%, such as more than 10%, or more than 20% or more than 50% or more than 70% by weight over the weight of the product) of short hydrocarbon chain alcohols such as aliphatic $C_1$-$C_4$ alcohols (e.g. ethanol or isopropanol). Without wishing to be bound by any theory, it is believed that short hydrocarbon chain alcohols might affect the microcapsule integrity thereby facilitating the leakage of the perfume content. This might explain why in certain examples disclosed herein below no noticeable difference in fragrance intensity was perceived by perfume evaluators between the test sample and the control.

Microcapsules amount into liquid household, laundry, personal care and cosmetic products may vary depending on several aspects such as the desired microcapsule concentration, the proportion of fragrance within the microcapsule and the amount of fragrance material necessary to create the olfactory effect desired. After removing all liquid components from a named product (i.e. measured as dry weight) the microcapsules of the invention can be present from 0.01 to 10% by weight, preferably from 0.05% to 2.5% by weight, more preferably from 0.1 to 1.25% by weight over the weight of the product. The microcapsules may be incorporated into the products by any conventional means usually as a liquid dispersion added at a suitable stage in the process but usually after any high shear mixing stage.

The abovementioned liquid products can conveniently be prepared by using an initial water-based dispersion comprising the microcapsules of the invention. This water-based dispersion (also referred to as slurry) functions thus as a concentrated fluid which is added to the above mentioned liquid products. Since this process entails a substantial dilution of the slurry components, microcapsules are contained in the slurry in amounts that are higher than the target amount in the final products. For this reasons, a slurry of the invention can contain microcapsules in amounts up to or above 30% (e.g. 40% or 50% or 60%) by weight over the weight of the slurry (wherein percentage is calculated as indicated above on the dry slurry).

The slurry can also conveniently be used as a storage medium for the microcapsules of the invention. In case the microcapsules are stored in the form of aqueous based slurry but no water (or a limited amount of water) must be present in the final product, the slurry can be spray-dried and the spray-dried microcapsules are then added to the final intended product.

In one embodiment, the present invention relates to the use of a polyethylenically unsatured monomer and which comprises one or more monomers selected from the group consisting of divinylbenzene, trivinylbenzene, and a methacrylic ester derived from polyhydric linear or branched $C_2$-$C_{24}$ alcohols or from linear or branched $C_2$-$C_{24}$ polyethylene glycols, to reduce leakage from a microcapsule comprising a core and a polymeric shell enclosing said core, wherein the core comprises a core material comprising an emulsifiable fragrance.

In one embodiment, the present invention relates to the use as presently defined wherein the monomer is not ethylene glycol dimethacrylate.

In one embodiment, the present invention relates to the use as presently defined wherein the microcapsule is as defined above.

In one embodiment, the present invention relates to the use as presently defined wherein the microcapsule is comprised in a solid powder composition as defined above.

In one embodiment, the present invention relates to the use as presently defined wherein the microcapsule is comprised in a product as defined above.

Further embodiments and advantages of the present invention will become apparent to a skilled reader in light of the examples provided below.

Composition of the fragrance material no. 1 (% by weight):

| | |
|---|---|
| Isobornyl acetate (CAS N ° 125-12-2): | 25 |
| Camphor gum powder synthetic (CAS N° 464-49-3): | 15 |
| Lilial (CAS N° 80-54-6): | 15 |
| Eucalyptol (CAS N° 470-82-6): | 8 |
| Ethyl- 2-methylpentanoate (CAS N° 39255-32-8): | 6 |
| Cedrol (CAS N° 77-53-2): | 6 |
| Allyl heptoate (CAS N° 142-19-8): | 5 |

(continued)

| | |
|---|---|
| Styrallyl acetate (CAS N° 93-92-5): | 5 |
| 2-Methylundecanal (CAS N° 110-41-8): | 5 |
| Vertenex (CAS N° 32210-23-4): | 5 |
| Coumarin (CAS N° 91-64-5): | 3 |
| Delta damascone (CAS N° 57378-68-4): | 2 |

The fragrance material1 contains 79% of bulky molecules.

Composition of the fragrance material no. 2 (% by weight):

| | |
|---|---|
| Allyl heptoate (CAS N° 142-19-8): | 12 |
| Verdox (CAS N° 88-41-5): | 12 |
| Lilial (CAS N° 80-54-6): | 10 |
| Gamma undecalactone (CAS N° 104-67-6): | 10 |
| Hexyl cinnamic aldehyde (CAS N° 101-86-0): | 10 |
| Ethylene brassylate (CAS N° 105-95-3): | 10 |
| Benzyl acetate (CAS N° 140-11-4): | 7 |
| Phenyl ethyl alcohol (CAS N° 60-12-8): | 6 |
| Ethyl- 2-methylpentanoate (CAS N° 39255-32-8): | 6 |
| Cyclamen aldehyde (CAS N° 103-95-7): | 5 |
| Camphor (CAS N° 76-22-2): | 3 |
| Diphenyl oxide (CAS: 101-84-8): | 2 |
| 2- methylundecanal (CAS N° 110-41-8): | 2 |
| Yara yara (CAS N ° 93-04-9): | 2 |

The fragrance material 2 contains 39% of bulky molecules.

Composition of the fragrance material no. 3 (% by weight):

| | |
|---|---|
| Cyclacet (CAS N ° 54830-99-8): | 22 |
| Verdox (CAS N ° 88-41-5): | 15.5 |
| Nerolin bromelia (CAS N° 93-18-5): | 14.5 |
| Isobornyl acetate (CAS N° 125-12-2): | 13.5 |
| Benzyl salicylate (CAS N° 118-58-1): | 10.5 |
| Lilial (CAS N° 80-54-6): | 7 |
| Fructone (CAS N° 6413-10-1): | 4 |
| Diphenyl oxide (CAS N° 101-84-8): | 2.5 |
| Isocyclo citral (CAS N° 1 335-66-6): | 2.5 |
| Camphor gum powder synthetic (CAS N° 464-49-3): | 2 |
| Eucalyptol (CAS N° 470-82-6): | 2 |
| Fruitate (CAS N° 80623-07-0): | 1.5 |
| Ethyl- 2-methylpentanoate (CAS N° 39255-32-8): | 15 |
| Delta damascone (CAS N° 57378-68-4): | 1 |

The fragrance material 3 contains 98.5% of bulky molecules.

Composition of the concentrated fabric softener (% by weight):

| | |
|---|---|
| Praepagen TQ 90 %: | 20.00 |
| Water: | 79.87 |
| Magnesium chloride hexahydrate 99%: Citric acid is added to get a pH=2.5 | 0.13 |

Example 1: preparation of capsules according to the invention (samples 1 to 3)

An aqueous phase was prepared by dissolving 4.0 g of poly(vinyl alcohol), hydrolyzed to 87-89 %, $M_w$=85000-124000 g/mol, in 196.0 g of water. An oil phase was prepared by mixing 85.0 g of fragrance material no. 1, 2 or 3, 13.7 g of 1,4-butane diol dimethacrylate, 13.1 g of methacrylic acid and 5.2 g of methyl methacrylate and 0.9 g of lauroyl peroxide. This mixture was stirred until complete dissolution of the lauroyl peroxide.

The aqueous phase and the oil phase were placed into a 500 mL-batch reactor equipped with a condenser, a thermometer, a nitrogen inlet and a deflocculating blade (diameter 4 cm). During all the process, the mixture was stirred at 900 rpm and nitrogen was bubbled through the mixture to remove oxygen. First, the mixture was heated from room temperature to 35 °C within 20 min and kept at 35 °C for 1 hour. The resultant emulsion was then heated to 70 °C within 30 min and kept at 70 °C for 4 hours. Finally, the resultant microcapsule dispersion was cooled to room temperature within 1 hour. The mean particle size of the resultant microcapsule dispersion was determined by laser diffraction (Volume median diameter (D(v, 0.5)).

Results:

| Sample number | Fragrance | Median volume diameter (D(v, 0.5)) - microns |
|---|---|---|
| 1 | 1 | 38.6 |
| 2 | 2 | 30.2 |
| 3 | 3 | 44.7 |

Example 2 (comparative): preparation of capsules using the crosslinker 1,4-butane diol diacrylate (samples 4 and 5)

An aqueous phase was prepared by dissolving 4.0 g of poly(vinyl alcohol), hydrolyzed to 87-89 %, $M_w$=85000-124000 g/mol, in 196.0 g of water. An oil phase was prepared by mixing 85.0 g of fragrance material no. 1 or 2, 13.7 g of 1,4-butane diol diacrylate, 13.1 g of methacrylic acid and 5.2 g of methyl methacrylate and 0.9 g of lauroyl peroxide. This mixture was stirred until complete dissolution of the lauroyl peroxide.

The aqueous phase and the oil phase were placed into a 500 mL-batch reactor equipped with a condenser, a thermometer, a nitrogen inlet and a deflocculating blade (diameter 4 cm). During all the process, the mixture was stirred at 900 rpm and nitrogen was bubbled through the mixture to remove oxygen. First, the mixture was heated from room temperature to 35 °C within 20 min and kept at 35 °C for 1 hour. The resultant emulsion was then heated to 70 °C within 30 min and kept at 70 °C for 4 hours. Finally, the resultant microcapsule dispersion was cooled to room temperature within 1 hour. The mean particle size of the resultant microcapsule dispersion was determined by laser diffraction (Volume median diameter (D(v, 0.5)).

Results:

| Sample number | Fragrance | Median volume diameter (D(v, 0.5)) - microns |
|---|---|---|
| 4 | 1 | 43.1 |
| 5 | 2 | 33.8 |

**Example 3 (comparative): preparation of a capsule using the crosslinker pentaerythritol triacrylate (sample 6)**

An aqueous phase was prepared by dissolving 4.0 g of poly(vinyl alcohol), hydrolyzed to 87-89 %, $M_w$=85000-124000 g/mol, in 196.0 g of water. An oil phase was prepared by mixing 85.0 g of fragrance material no. 2, 13.7 g of pentaerythritol triacrylate, 13.1 g of methacrylic acid and 5 .2 g of methyl methacrylate and 0.9 g of lauroyl peroxide. This mixture was stirred until complete dissolution of the lauroyl peroxide.

The aqueous phase and the oil phase were placed into a 500 mL-batch reactor equipped with a condenser, a thermometer, a nitrogen inlet and a deflocculating blade (diameter 4 cm). During all the process, the mixture was stirred at 900 rpm and nitrogen was bubbled through the mixture to remove oxygen. First, the mixture was heated from room

temperature to 35 °C within 20 min and kept at 35 °C for 1 hour. The resultant emulsion was then heated to 70 °C within 30 min and kept at 70 °C for 4 hours. Finally, the resultant microcapsule dispersion was cooled to room temperature within 1 hour. The resultant microcapsule dispersion has a mean particle size of 38.7 μm (Median volume diameter (D (v, 0.5)) determined by laser diffraction).

**Example 4 (comparative): preparation of a capsule using two crosslinkers: 1,4-butanediol diacrylate and pentaerythritol triacrylate (sample 7)**

An aqueous phase was prepared by dissolving 4.0 g of poly(vinyl alcohol), hydrolyzed to 87-89 %, $M_w$=85000-124000 g/mol, in 196.0 g of water. An oil phase was prepared by mixing 85.0 g of fragrance material no. 2, 10.3 g 1,4-butanediol diacrylate, 3.4 g pentaerythritol triacrylate13.1 g of methacrylic acid and 5 .2 g of methyl methacrylate and 0.9 g of lauroyl peroxide This mixture was stirred until complete dissolution of the lauroyl peroxide.

The aqueous phase and the oil phase were placed into a 500 mL-batch reactor equipped with a condenser, a thermometer, a nitrogen inlet and a deflocculating blade (diameter 4 cm). During all the process, the mixture was stirred at 900 rpm and nitrogen was bubbled through the mixture to remove oxygen. First, the mixture was heated from room temperature to 35 °C within 20 min and kept at 35 °C for 1 hour. The resultant emulsion was then heated to 70 °C within 30 min and kept at 70 °C for 4 hours. Finally, the resultant microcapsule dispersion was cooled to room temperature within 1 hour. The resultant microcapsule dispersion has a mean particle size of 38.9 μm (Median volume diameter (D (v, 0.5)) determined by laser diffraction).

**Example 5 (comparative): preparation of a capsule of smaller size than according to the invention (sample 8)**

An aqueous phase was prepared by dissolving 4.0 g of poly(vinyl alcohol), hydrolyzed to 87-89 %, $M_w$=85000-124000 g/mol, in 196.0 g of water. An oil phase was prepared by mixing 85.0 g of fragrance material no. 2, 13.7 g of 1,4-butane diol dimethacrylate, 13.1 g of methacrylic acid and 5.2 g of methyl methacrylate and 0.9 g of lauroyl peroxide. This mixture was stirred until complete dissolution of the lauroyl peroxide.

The aqueous phase and the oil phase were poured into a 1 L-beaker placed in an ice-water bath and emulsified together using a high-speed stirrer (Dispermix, laboratory series X10, Ystral) at 7000 rpm for 2 min. At this stage, the emulsion had a mean particle size of 2.4 μm (Median volume diameter (D(v, 0.5)) determined by laser diffraction). The emulsion was then transferred into a 500 mL-batch reactor equipped with a condenser, a thermometer, a nitrogen inlet and a deflocculating blade (diameter 4 cm). During all the process, the mixture was stirred at 900 rpm and nitrogen is bubbled through the mixture to remove oxygen. First, the mixture was heated from room temperature to 35 °C within 20 min and kept at 35 °C for 1 hour. The resultant emulsion was then heated to 70 °C within 30 min and kept at 70 °C during 4 hours. Finally, the resultant microcapsule dispersion was cooled to room temperature within 1 hour. The resultant microcapsule dispersion has a mean particle size of 2.9 μm (Median volume diameter (D(v, 0.5)) determined by laser diffraction).

**Example 6: preparation of capsules according to the invention (samples 9 to 12)**

An aqueous phase was prepared by dissolving 4.0 g of poly(vinyl alcohol), hydrolyzed to 87-89 %, $M_w$=85000-124000 g/mol, in 196.0 g of water. An oil phase was prepared by mixing 85.0 g of fragrance material no. 2, 13.7 g of specific monomer (II) (for sample definition see table below with median volume diameters), 13.1 g of methacrylic acid, 5 .2 g of methyl methacrylate and 0.9 g of lauroyl peroxide. This mixture was stirred until complete dissolution of the lauroyl peroxide.

The aqueous phase and the oil phase were placed into a 500 mL-batch reactor equipped with a condenser, a thermometer, a nitrogen inlet and a deflocculating blade (diameter 4 cm). During all the process, the mixture was stirred at 900 rpm and nitrogen was bubbled through the mixture to remove oxygen. First, the mixture was heated from room temperature to 35 °C within 20 min and kept at 35 °C for 1 hour. The resultant emulsion was then heated to 70 °C within 30 min and kept at 70 °C for 4 hours. Finally, the resultant microcapsule dispersion was cooled to room temperature within 1 hour. The mean particle size of the resultant microcapsule dispersion was determined by laser diffraction (Volume median diameter (D(v, 0.5)).

Results:

| Sample number | monomer (II) | Median volume diameter (D(v, 0.5)) - microns |
|---|---|---|
| 9 | Ethylene glycol dimethacrylate | 35.3 |
| 10 | 1,3-propane diol dimethacrylate | 41.5 |
| 11 | Di(ethylene glycol) dimethacrylate | 35.8 |
| 12 | Ethoxylated pentaerythritol tetramethacrylate | 45.9 |

**Example 7: fragrance leakage during storage in fabric softener**

The fragrance released in the fabric softener was determined through extraction with solvent and analysis by gas chromatography. The fragrance leakage is the ratio of fragrance released in the fabric softener to the encapsulated fragrance.

Details of the method:

Procedure:

A mixture containing 0.5 % w/w of slurry dispersion and 99.5 % w/w of concentrated fabric softener was stored in a glass bottle in an oven at the controlled temperature of 40 °C for 1/2/4/6 weeks. After each time of storage, the mixture was shaken and 10 g are withdrawn. This sample was centrifuged to separate the fabric softener from the capsules. 1 g of centrifuged fabric softener was mixed with 1 g of Celite, 545. 5 mL of pentane and 50 μL of an internal standard solution are added. The mixture was agitated on a roller bed for 1 hour. The supernatant was then injected in GC/FID (gas chromatography apparatus using a flame ionization detector). Integration areas were determined from the FID signal using Agilent Chemstation software. Each extract was analyzed three times. The internal standard solution was a solution of methyl decanoate in hexane at a concentration of 10mg/mL.

Instrumentation:

Agilent 6890 GC connected to Chemstation software
Column: HP-SMS, 30m x 0.25mm x 0.25μm
Oven temperature: 50°C for 2min then heat to 280°Cat 10°C/min and hold at 280°C for 5min. Injector: 250°C, Detector: 250°C
2μL injection volume (splitless)

Calculations:

Determination of the weight of leaked fragrance component i in the sample:

$$W_{perf,i} = \frac{A_{perf,i} \times w_{IS}}{A_{IS}}$$

$W_{perf,i}$: weight of leaked fragrance component i (mg)
$A_{perf,i}$: fragrance component i area
$W_{IS}$: weight of internal standard (mg)
$A_{IS}$: internal standard area
Determination of the weight of leaked fragrance in the sample:

$$W_{frag} = \sum_i W_{perf,i}$$

$W_{frag:}$ weight of leaked fragrance (mg)
Determination of the theoretical weight of fragrance in the sample for a total leakage:

$$W_{frag-theo} = \frac{\%\,frag\ slurry \times \%\,slurry}{W_{softener} \times 100}$$

$W_{frag\text{-}theo}$: theoretical weight of fragrance in sample for a total leakage (mg)
%frag slurry: percentage of fragrance in slurry
%slurry: percentage of slurry in softener
$W_{softener}$: softener weight for extraction (mg)

Determination of the theoretical weight of fragrance component i in the sample for a total leakage:

$$W_{perf-theo} = \frac{\%\,frag\ slurry \times \%\,perf\ ,i \times \%\,slurry}{W_{softener} \times 100}$$

$W_{perf\text{-}theo}$: theoretical weight of fragrance component i in sample for a total leakage (mg) %slurry: percentage of slurry in softener
% perf, i: percentage of fragrance component i in fragrance

Determination of the percentage of the fragrance leakage:

$$\%leakage_{frag} = \frac{W_{frag}}{W_{frag-theo}} \times 100$$

$\%leakage_{frag}$: percentage of fragrance leakage

Determination of the percentage of leakage of fragrance component i:

$$\%leakage_{perf} = \frac{W_{perf}}{W_{perf-theo}} \times 100$$

$\%leakage_{perf}$: percentage of leakage of fragrance component i:

<u>Results:</u>

Results for capsules containing the fragrance **<u>material no.</u>** <u>1:</u>

| Time of storage | Fragrance leakage (%) (Standard deviation %) | | |
|---|---|---|---|
| | Sample 1 | Sample 4 | Sample 8 |
| 1 week at 40 °C | 9.8 (0.52) | 26.4 (0.46) | n.d |
| 2 weeks at 40 °C | 13.0 (0.33) | 34.2 (0.16) | 55.6 (0.21) |
| 4 weeks at 40 °C | 17.2 (0.23) | 38.1 (0.42) | n.d |
| 6 weeks at 40 °C | 16.1 (0.33) | 46.2 (0.95) | n.d |
| n.d: not determined | | | |

Results for capsules containing the fragrance **<u>material no.</u>** 2:

| Time of storage | Fragrance leakage (%) (Standard deviation %) | | | |
|---|---|---|---|---|
| | Sample 2 | Sample 5 | Sample 6 | Sample 7 |
| 1 week at 40 °C | 42.0 (0.95) | 59.7 (0.61) | 73.0 (2.27) | 48.0 (0.38) |
| 2 weeks at 40 °C | 44.7 (0.72) | 67.0 (0.37) | 76.4 (2.58) | 54.9 (0.62) |

(continued)

| Time of storage | Fragrance leakage (%) (Standard deviation %) | | | |
|---|---|---|---|---|
| | Sample 2 | Sample 5 | Sample 6 | Sample 7 |
| 4 weeks at 40 °C | 49.0 (0.76) | 76.4 (1.43) | 77.7 (1.32) | 61.2 (0.77) |

| Time of storage | Fragrance leakage (%) (Standard deviation %) | | | |
|---|---|---|---|---|
| | Sample 9 | Sample 10 | Sample 11 | Sample 12 |
| 1 week at 40 °C | 40.3 (1.46) | 51.5 (0.78) | 54.6 (1.84) | 68.2 (0.42) |
| 2 weeks at 40 °C | 42.5 (1.24) | 58.4 (1.14) | 61.0 (1.36) | 75.3 (0.05) |
| 4 weeks at 40 °C | 47.4 (0.51) | 59.0 (0.43) | 68.6 (1.88) | 74.9 (0.39) |

It is clearly evident that the capsules according to the invention (samples 1 and 2 and 9 to 12) had in most cases a reduced leakage in comparison to other capsules. Additionally, the results obtained when variability linked to fragrance choice is made irrelevant, support a preference for the use of 1,4-butylene glycol dimethacrylate; 1,3-propylene glycol dimethacrylate, ethylene glycol dimethacrylate or diethylene glycol dimethacrylate; in particular 1,4-butylene glycol dimethacrylate or ethylene glycol dimethacrylate.

Results of leakage of different molecules from the capsules of sample 1:

| Time of storage | Leakage of fragrance components (%) (Standard deviation %) | | | |
|---|---|---|---|---|
| | Isobornyl acetate | Verdox | Ethyl- 2-methylpentanoate | Allyl heptoate |
| 1 week at 40 °C | 1.6 (0.21) | 2.1 (0.22) | 47.2 (2.68) | 59.9 (2.24) |
| 2 weeks at 40 °C | 2.1 (0.11) | 1.7 (0.26) | 54.2 (1.35) | 70.2 (2.53) |
| 4 weeks at 40 °C | 2.8 (0.18) | 3.3 (0.43) | 77.5 (2.94) | 85.6 (2.89) |
| 6 weeks at 40 °C | 3.0 (0.03) | 3.6 (0.64) | 74.6 (3.65) | 79.7 (1.04) |

It is clearly evident that the bulky molecules according to the invention (isobornyl acetate and Verdox) had a reduced leakage in comparison to other molecules (Ethyl- 2-methylpentanoate and allyl heptoate).

**Example 8: olfactory performances of capsules when used in fabric softener**

Preparation of the fabric softener formulation containing capsules:

A mixture containing 0.5 % w/w of slurry dispersion and 99.5 % w/w of concentrated fabric softener described above was prepared. This mixture was stored at 40 °C for a defined period of time.

Washing procedure:

Cotton toweling mitts and towels prewashed with an unperfumed liquid detergent at 90°C, 16°dH, were placed in a Miele washing machine. The total ballast was 2.0 kg. A washing cycle was performed at 40 °C (spin drying: 900 rpm). 40 mL of fabric softener formulation was added during the rinsing process. The cotton gloves were dried for 24 hours at room conditions. The effect of the scent was then assessed as a blind experiment in a sensory manner before and after they were rubbed against hands. Scores were given from 0 (non-noticeable scent) to 5 (very strong scent).

Results:

| Time of storage at 40 °C (weeks) | Olfactory performances (Before rubbing-After rubbing) | | | | | | |
|---|---|---|---|---|---|---|---|
| | Sample 1 | Sample 2 | Sample 3 | Sample 4 | Sample 5 | Sample 6 | Sample 7 |
| 0 | 0.5-5.0 | 0.25-4.5 | 0-4.0 | 0-1.5 | 0-1.0 | 0-1.0 | 0.25-4.5 |
| 1 | 0.5-4.5 | 0-2.5 | n.d. | 0-0.5 | 0-0.5 | 0-0.5 | 0-2.0 |
| 2 | 0-3.5 | 0-2.0 | n.d. | 0-0 | 0-0.5 | 0-0 | 0-1.5 |
| 4 | 0-3.5 | 0-2.0 | 0.5-2.5 | 0-0 | 0-0 | 0-0 | 0-1.0 |
| 6 | 0-3.5 | n.d. | n.d. | 0-0 | n.d. | n.d. | n.d. |
| n.d: not determined | | | | | | | |

The results of example 8 show that microcapsules of the invention provided for long-lasting olfactory performances. Due to reduced leakage, after four or six weeks a noticeable scent could still be perceived. A comparison of samples 2 and 7 is particularly meaningful to show superiority of the invention since both samples contained the same fragrance material (material no. 2) and both samples provided the same initial scent perception. Scores obtained by samples 4, 5 and 6 were less meaningful. Possible explanations are that in these samples the microcapsule shells were thicker or harder to break than the shells of the other samples, thus leading to inferior scent perception after rubbing.

**Example 9: study of the visibility of the capsules on fabric squares**

For this study, slurry dispersions were prepared with the following average capsule diameters: 5.7, 10.4, 19.9, 31.8, 38.3 and 59.6 μm (as measured by the light scattering method described above). Dispersions containing 0.4 % by weight of capsules in deionized water were prepared. About 1.3 g of these dispersions was sprayed on the entire surface (10x12 cm) of two different types of dark flat sheet fabrics:

- Knitted cotton fabric colored with Reactive Black 5 dye (6% dye by weight of fabric)
- Black cotton denim (Levi® 501)

After spraying, the fabric squares were dried at room temperature during 20 min. Visual inspection of the fabric squares was performed separately as a blind experiment by three different persons in a color viewing booth (standard daylight illuminant D65). Untreated fabric squares were used as blank samples. Residues on the material clothes were only clearly visible in the case of the microcapsules with an average capsule diameter of 59.6 éμm.

Personal Care and Cosmetic Examples

The ability of the microcapsule dispersion of sample 1 (as obtained in Example 1) to release the fragrance during the application and over time (boost and long-lasting effects) was determined by evaluating the fragrance odour intensity give off at time of use and retained on hair or skin washed or painted with different personal care compositions comprising the capsule dispersion compared with the free fragrance at the same concentration. Odor intensity was assessed by two trained fragrance evaluators. INCI = International Nomenclature of Cosmetic Ingredients

**Example 10: Preparation and evaluation of capsules-containing shower gel**

A test sample containing capsules sample 1 was prepared by adding 0.5g of the capsule slurry to 49.5 g of shower gel base having the formulation of table 1. The capsules were homogeneously distributed in the base, as observed by optical microscopy. A control sample was created by admixing 0.133g of the free fragrance material no. 1 (fragrance material no. 1 is the fragrance used in capsule slurry sample 1 and the capsule slurry contains 27% of this fragrance) into 49.867g of the same shower gel. Samples were left for 24h at room temperature and thoroughly mixed just before testing.

| **Materials** | **INCI** | **% w/w** |
|---|---|---|
| Water | Water | Qsp 100 |
| Texapon N70 | Sodium lauryl ether sulphate | 14.30 |

(continued)

| Materials | INCI | % w/w |
|---|---|---|
| Pricerine 9091 | Glycerin | 1.80 |
| Dehyton AB 30 | Cocobetaine | 6.00 |
| Tegin BL 315 | Glycol distearate | 1.20 |
| Merquat S | Polyquaternium-7 | 1.10 |
| Miranol C2M | Disodium cocoamphodiacetate | 1.55 |
| Carbopol Aqua-SF1 | Acrylate copolymer | 1.00 |
| Procetyl AWS | PPG-5 Ceteth-20 | 0.90 |
| Comperlan | Cocamide monethanolamide | 0.8 |
| Sodium chloride | Sodium chloride | 1.00 |
| Sodium hydroxide (30%) | Sodium hydroxide | 0.33 |
| Nipaguard DMDMH | DMDM Hydantoin | 0.3 |
| Dissolvine Na 2 | Disodium EDTA | 0.25 |
| Nipagin M | Methyl paraben | 0.10 |
| Nipagin M sodium | Sodium methyl paraben | 0.10 |
| Citric acid | Citric acid | pH = 5.7 |
| Table 1: Shower gel formula | | |

Test Method: The panellist wetted his/her hands for 5 sec with running tap water at 37°C then 1,5 ml of shower gel was applied by syringe into the hands. The panellist washed his hands then rinsed them for 15 sec under tap water at 37°C and the excess water removed with a paper towel. Two trained perfume evaluators assessed the odour intensity. Three hours later the skin was rubbed slightly and the perfume intensity re-assessed.

The evaluation compared the capsule containing sample to the control sample containing free fragrance. There was no significant difference in strength during washing. However the fragrance was stronger for the capsule containing sample when the skin was rubbed 3 hours after drying.

**Example 11: Preparation and evaluation of capsules-containing leave-on hair conditioner**

A test sample containing capsules sample 1 was prepared by adding 0.5g of the capsule slurry to 49.5 g of leave on hair conditioner base having the formulation of table 2. The capsules were homogeneously distributed in the base, as observed by optical microscopy. A control sample was created by admixing 0.133g of the free fragrance material no. 1 (fragrance material no. 1 is the fragrance used in capsule slurry sample 1 and the capsule slurry contains 27% of this fragrance) into 49.867g of the same conditioner base. Samples were left for 24h at room temperature and thoroughly mixed just before testing.

| Materials | INCI | % w/w |
|---|---|---|
| Water | Water | Qsp 100 |
| Incroquat Behenyl TMC-25 | Ceterayl alcohol / Behentrimonium chloride | 4.0 |
| DC 2-8566 AMINO FLUID | Amodimethicone | 2.0 |
| Natrosol 250H | Hydroxycellulose | 1.2 |
| DC 556 | Phenyl trimethicone | 1.0 |
| Cropeptide W PF | Hydrolysed wheat protein / Hydrolysed wheat starch | 1.0 |
| Dowanol EPH | Phenoxyethanol | 0.2 |

(continued)

| Materials | INCI | % w/w |
|---|---|---|
| Citric acid | Citric acid | pH = 4.4 |
| Table 2: Leave-on conditioner formula | | |

A hair swatch of approximately 10g weight was combed and dipped into water (300mL at 37°C) for 15 seconds. Excess water is removed by twice drawing the hair swatch through two fingers to act as a squeegee. 0.4 ml of the leave-on conditioner were applied by syringe onto the wet hair swatch. The hair swatch was massaged by hand with the leave-on conditioner for 30 sec and perfume intensity was assessed by two trained perfume evaluators. Then the samples were air-dried at ambient temperature for 3 hours by hanging from a frame. After 3 hours the hair was dry and the perfume intensity was assessed. Then the swatch was combed 3 times and the intensity assessed again. After 24 hours hanging at ambient temperature the same swatch was assessed again, before and after combing by two trained perfume evaluators. Hair swatches treated with the leave-on conditioner sample comprising the capsule dispersion were compared to a control sample containing the free fragrance.

The fragrance was stronger at the time of use for the conditioner containing capsules. Also when hair was combed 3 and 24 hours after rinsing the sample containing the capsule dispersion gave a stronger fragrance. These effects are still repeatable on samples stored at 45°C after 2, 4 and 8 weeks showing that the capsubs retain the perfume.

**Example 12: Preparation and evaluation of capsules-containing stick deodorant**

A test sample containing capsules sample 1 was prepared by adding 0.5g of the capsule slurry to 49.5 g of stick deodorant base having the formulation of table 3. The capsules were homogeneously distributed in the base, as observed by optical microscopy. A control sample was created by admixing 0.133g of the free fragrance material no. 1 into 49.867g of the same stick deodorant base. Samples were left for 24h at room temperature before testing.

Table 3: Stick deodorant formula

| Materials | INCI | % w/w |
|---|---|---|
| DC 245 Fluid | Cyclopentasiloxane | 22.0 |
| Ach 331 | Aluminium chlorhydrate | 15.0 |
| Lanette-18 | Stearyl alcohol | 16 |
| Silkfo 366 NF | Hydrogenated polydecene | 15 |
| Crodamol IPP | Isopropyl palmitate | 13.5 |
| Arlamol PB14-LQ-(RB) | PPG-14 Butyl ether | 11.0 |
| Cutina HR pulver | Hydrogenated castor oil | 5.0 |
| DUB DS PEG 8 | PEG-8 Distearate | 2.5 |

Test Method: 0.3 grams of each stick deodorant was applied onto cellulose fragrance blotter and the blotter was air-dried during 3 hours in ambient conditions in a test room. The perfume intensity was assessed by two trained perfume evaluators before and after rubbing the blotter. After rubbing, there was a noticeably stronger fragrance for the blotter treated with the sample containing a capsule dispersion compared with the control sample. Repeating the experiment with samples which had been stored at 45°C for 2 weeks and 4 weeks also gave stronger fragrances for the samples containing capsules.

**Example 13: Preparation and evaluation of capsules-containing roll-on deodorant**

A test sample containing capsules sample 1 was prepared by adding 0.5g of the capsule slurry to 49.5 g of roll-on deodorant base having the formulation of table 4. The capsules were homogeneously distributed in the base, as observed by optical microscopy. A control sample was created by admixing 0.133g of the free fragrance Material no. 1 into 49.867g of the same roll-on deodorant. Samples were left for 24h at room temperature and thoroughly mixed just before testing.

Table 4: Roll-on deodorant formula

| Materials | INCI | % w/w |
|---|---|---|
| Water | Water | Qsp 100 |
| Ach 330 solution | Aluminium chlorhydrate | 27.5 |
| Nafol 1618 F | Cetearyl alcohol | 2.5 |
| Simulsol CS | Ceteareth-33 | 1.25 |
| DC 200 fluid 350 cst | Dimethicone | 0.5 |

Test method: 0.3 grams of a roll-on deodorant were applied onto a cellulose blotter and the blotter was air-dried for 3 hours. The perfume intensity was assessed by two trained perfume evaluators before and after rubbing the blotter 10 minutes after application (when the blotter was still slightly wet) and 3 hours after application (when the blotter was totally dry).

There was a noticeably stronger fragrance for the test sample containing the capsule dispersion compared with the control sample at all assessment points. Repeating the experiment using samples of roll-on deodorant which had been stored at 45°C.for 2 weeks, 4 weeks, and 8 weeks showed a stronger fragrance after 3 hours drying for the product containing the capsules.

**Example 14: Preparation and evaluation of capsules-containing aerosol antipersairant/deodorant**

A test sample containing capsules sample 1 was prepared by adding 0.5g of the capsule slurry to 49.5 g of aerosol deodorant base having the formulation of table 5. The capsules were homogeneously distributed in the base. A control sample was created by admixing 0.133g of the free fragrance material no. 1 into 49.867g of the same aerosol deodorant base. Samples were left for 24h at room temperature and thoroughly mixed just before testing.

Table 5: Aerosol deodorant formula

| Materials | INCI | % w/w |
|---|---|---|
| Isobutane | Isobutane | Qsp 100 |
| Reach 103 | Aluminium chlohydrate | 3.25 |
| Xiameter PMX 200 silicone 10 cs | Dimethicone | 5.55 |
| DC 1503 fluid | Dimethicone / Dimethiconol | 2.15 |
| Citroflex 2 | Triethyl citrate | 1.55 |
| Crodamol IPP | Isopropyl palmitate | 0.9 |
| Optimal 2550 OR | Perlite | 0.20 |
| Tixogel MP 250 | Stearalkonium bentonite | 0.40 |

Test Method: 2 sprays of aerosol antiperspirant were applied onto blotters depositing approximately 0.2g of product and the blotters were allowed to dry in ambient air for 2 minutes. The perfume intensity was assessed by two trained perfume evaluators before and after rubbing the blotter.

There was a noticeably stronger fragrance for the sample containing the capsule dispersion compared with the control sample after rubbing. Repeating the experiment on samples of product stored for 2 weeks at 45°C again gave a stronger fragrance for the product containing capsules after rubbing.

**Example 15: Preparation and evaluation of capsules-containing body lotion**

A test sample containing capsules sample 1 was prepared by adding 0.25g of the capsule slurry to 49.75g of body lotion base having the formulation of table. The capsules were homogeneously distributed in the base, as observed by optical microscopy. A control sample was created by admixing 0.067g of the free fragrance Material no. 1 into 49.933 g of the same body lotion base. Samples were left for 24h at room temperature and thoroughly mixed just before testing.

Table 6: body lotion formula

| Materials | INCI | % w/w |
|---|---|---|
| Water | Water | Qsp 100 |
| Emulgade CM | Cetearyl Isononanoat / Ceteareth-20 /Cetearyl Alcohol / Glyceryl Stearate / Glycerin / Ceteareth-12 / Cetyl Palmitate | 10.0 |
| Mineral oil | Mineral oil | 8.0 |
| Pricerine 9091 | Glycerin | 5.0 |
| Xiameter PMX 345 | Cyclopentasiloxane / Cyclohexasiloxane | 3.0 |
| Cetiol PGL | Hexyldecanol / Hexyldecyl laurate | 0.75 |
| Carbopol ultrez 10 | Carbomer | 0.25 |
| Kathon CG | Methylchloroisothiazolinone / Methylisothiazolinone | 0.05 |
| Sodium hydroxide | Sodium hydroxyide | pH = 6.0 |

Test method: 0.2 ml of the body lotion were applied by syringe on the back of the hand and spread for 10 seconds. Then the skin was rubbed slightly 3 hours after application. The perfume intensity was assessed by two trained perfume evaluators at the time of use and before and after rubbing the skin. The skin areas were treated with the body lotion sample comprising the capsule dispersion and compared to the control sample containing the free fragrance.

There was a noticeable benefit for sample containing capsule dispersion compared with the control sample. The odour intensity was higher after rubbing both at time of use and 3 hours after application for the sample containing capsule dispersion. Repeating the experiment on samples of products which had been stored at 45°C br 2 weeks, 4 weeks and 8 weeks also gave a noticeable boost after rubbing at both assessment points for the product containing the capsules.

**Example 16: Preparation and evaluation of capsules-containing make-up remover**

A test sample containing capsules sample 1 was prepared by adding 0.5g of the capsule slurry to 49.5 g of make-up remover base having the formulation of table 7. The capsules were homogeneously distributed in the base, as observed by optical microscopy. A control sample was created by admixing 0.133g of the free fragrance material no. 1 into 49.867g of the same make-up remover base. Samples were left for 24h at room temperature and thoroughly mixed just before testing.

Table 7: Make-up remover formula

| Materials | INCI | % w/w |
|---|---|---|
| Water | Water | Qsp 100 |
| Emulgade CM | Cetearyl Isononanoat / Ceteareth-20 /Cetearyl Alcohol / Glyceryl Stearate / Glycerin / Ceteareth-12 / Cetyl Palmitate | 10.0 |
| Pricerin 9091 | Glycerin | 2.0 |
| Sepicid HB | Phenoxyethanol / Methylparaben / Ethylparaben / Propylparaben / Butylparaben | 0.5 |
| Triethanolamine | Triethanolamine | pH = 7.2 |

Test method: 2 ml of the make-up remover were applied by pipette on a cotton disc. The product was wiped onto the back of the hand of a volunteer from the cotton disc for 10 seconds. Perfume intensity was assessed at the time by two trained perfume evaluators. Three hours after application the perfume intensity above the area treated was again assessed by the evaluators, then the skin was rubbed lightly and the fragrance intensity re-assessed.

There was a noticeably stronger perfume for the test sample containing the capsule dispersion compared with the control sample both at the time of application and later when the skin was rubbed 3 hours after application. Repeating the experiment on samples of products which had been stored at 45°C for 2 weeks again gave strongerperfume at both assessment times for the product containing capsules.

**Example 17: Preparation and evaluation of capsules-containing sunscreen lotion**

A test sample containing capsules sample 1 was prepared by adding 0.5g of the capsule slurry to 49.5 g of sunscreen lotion base having the formulation of table 8. The capsules were homogeneously distributed in the base, as observed by optical microscopy. A control sample was created by admixing 0.133g of the free fragrance material no. 1 into 49.867g of the same sunscreen lotion. Samples were left for 24h at room temperature and thoroughly mixed just before testing.

Table 8: Sunscreen spray formula

| **Materials** | **INCI** | **% w/w** |
|---|---|---|
| Water | Water | Qsp 100 |
| Uvinul MC 80 | Ethylhexyl methoxycinnamate | 8.0 |
| Crodamol GTCC-LQ | Caprylic / Capric triglyceride | 7.0 |
| Montanov L | C14-22 Alkylalcohol and C12-20 Alkylglucoside | 2.5 |
| Eusolex 9020 | Butyl methoxydibenzoymethane | 1.0 |
| Sepicide HB | Phenoxyethanol / methHENOXYETHANOL / Phenoxyethanol / Methylparaben / Ethylparaben / | 1.0 |
| Simulgel EG | Sodium acrylate / Sosium acryloyl dimethyl taurate copolymer / Isohexadecane / Polysorbate 80 | 0.7 |
| EDTA B pulver | Tetrasodium EDTA | 0.05 |
| DL alpha tocopherol | Tocopherol | 0.05 |

Test Method: approximately 0.4 grams of the sunscreen was sprayed on the back of the hand of a volunteer from a distance of 10cm and spread for 10 seconds. Three hours after application the perfume intensity above the area treated was assessed by two trained perfume evaluators, then the skin was rubbed lightly and the fragrance intensity re-assessed.

There was a noticeably stronger fragrance for the test sample containing the capsule dispersion compared with the control sample when the skin was rubbed 3 hours after application.

**Example 18: Preparation and evaluation of capsules-containing setting lotion**

A test sample containing capsules sample 1 was prepared by adding 0.5g of the capsule slurry to 49.5 g of hair setting lotion base having the formulation of table 9. The capsules were homogeneously distributed in the base, as observed by optical microscopy, and the base became cloudy. A control sample was created by admixing 0.133g of the free fragrance material no. 1 into 49.867g of the same setting lotion. Samples were left for 24h at room temperature and thoroughly mixed just before testing.

Table 9: Setting lotion formula

| **Materials** | **INCI** | **% w/w** |
|---|---|---|
| Water | Water | Qsp 100 |
| Luviskol 30 | PVP | 16.7 |
| Propylene glycol | Propylene glycol | 3.0 |
| Cremophor RH 40 | PEG-40 hydrogenated castor oil | 1.0 |
| Fixate plus | Polyacrylate-14 | 0.98 |
| Phenoxethol | Phenoxyethanol | 0.5 |
| Polyglykol 2000S | PEG-40 | 0.3 |
| D-Panthenol 75W | Panthenol | 0.2 |
| Triethanolamine | Triethanolamine | 0.12 |

(continued)

| Materials | INCI | % w/w |
|---|---|---|
| Nipagin M | Methylparaben | 0.1 |

Test Method: Approximately 2.4 grams of each setting lotion sample was sprayed onto wet hair swatches from a distance of 10cm. The hair swatches were rubbed slightly by hand with the setting lotion for 30 sec each (15s on both sides) then air-dried. 3 hours and 24 hours after application the hair swatches were combed 3 times. The perfume intensity was assessed by two trained perfume evaluators at time of use, then before and after combing after 3 and 24 hours.

There was a noticeably stronger perfume for the test sample containing the capsule dispersion compared with the control sample at time of use and when hair was combed after 3 hours and 24 hours. Repeating the experiment on samples of products that have been stored at 45°C for 2 weeks and 4 weeks again gave stronger fragrance after combing 3 hours and 24 hours after treatment.

**Example 19: Preparation and evaluation of capsules-containing hair gel**

A test sample containing capsules sample 1 was prepared by adding 0.25g of the capsule slurry to 49.5 g of hair gel base having the formulation of table 10 and making up to 50g with water. The capsules were homogeneously distributed in the base, as observed by optical microscopy, and the base became cloudy. A control sample was created by admixing 0.067g of the free fragrance material no. 1 into 49.933g of the same hair gel base. Samples were left for 24h at room temperature and thoroughly mixed just before testing.

Table 10: Hair gel formula

| Materials | INCI | % w/w |
|---|---|---|
| Water | Water | Qsp 100 |
| Luviset clear | VP / methacrylamide / N-vinyl imidazole copolymer | 15.0 |
| Triethanolamine | Triethanolamine | 0.6 |
| Carbopol ultrez 21 | Acrylates / C10-30 alkylacrylate crosspolymer | 0.40 |
| Uvinul P25 | PEG-25 PABA | 0.05 |
| Kathon CG | Methylchloroisothiazolinone / Methylisothiazolinone | 0.05 |

Test Method: 2.4 ml of the hair gel were applied by syringe onto wet hair swatches. The hair swatches were smoothed by hand with the hair gel for 20 sec each (10s on both sides) then air-dried. Three hours and 24 hours after application the perfume intensity was assessed by two trained perfume evaluators before and after combing. Hair swatches were treated with the hair gel sample comprising the capsule dispersion compared to the control sample containing the free fragrance.

The fragrance was stronger for sample containing the capsule dispersion compared with the control sample after combing the hair 3 hours after application and after combing 24 hours after application. Repeating the experiment on samples of product that had been stored at 45°C for 2, 4 and 8 weeks again gave stronger perfume at the 3 and 24 hours assessment points after combing, for the product containing capsules.

**Example 20: Preparation and evaluation of capsules-containing shampoo**

A test sample containing capsules sample 1 was prepared by adding 0.5g of the capsule slurry to 49.5 g of shampoo base having the formulation of table 11. The capsules were homogeneously distributed in the base, as observed by optical microscopy. A control sample was created by admixing 0.133g of the free fragrance material no. 1 into 49.867g of the same shampoo base. Samples were left for 24h at room temperature and thoroughly mixed just before testing.

Table 11: Shampoo formula

| Materials | INCI | % w/w |
|---|---|---|
| Water | Water | Qsp 100 |

(continued)

| Materials | INCI | % w/w |
|---|---|---|
| Texapon N70 | Sodium lauryl ether sulphate | 13.8 |
| Miranol C2M | Disodium cocoamphodiacetate | 7.6 |
| Euperlan PK 3000 | Glycol distearate / Laureth-4 / Cocamidopropylbetain | 1.8 |
| Sodium chloride | Sodium chloride | 1.2 |
| Tegosoft GC | PEG-7 Glyceryl cocoate | 1.0 |
| Sodium benzoate | Sodium benzoate | 0.5 |
| D Panthenol 75L | Pantenol | 0.5 |
| Celquat SC 230M | Polyquaternium-10 | 0.35 |
| Niacinamide | Niacinamide | 0.2 |
| Salicylic acid | Salicylic acid | 0.2 |
| Citric acid | Citric acid | pH = 5.0 |

Test Method: 2 ml of the shampoo were applied by syringe onto wet hair swatches. The hair swatches are rubbed by hand for 60 seconds each then rinsed for 60s with stationary shower rinse at 37°C with no manipulation of hair swatches Then the hair swatches were air-dried and combed 3 times 3 hours and 24 hours after application. The perfume intensity was assessed by two trained perfume evaluators at time of use wet and just dry then before and after combing after 3 and 24 hours.

The perfume was stronger when the hair was combed 3 and 24 hours after treatment when dry.

**Example 21: Preparation and evaluation of capsules-containing setting lotion with alcohol**

A dispersion containing capsules sample 1 was mixed at 0,5% by weight in an alcoholic setting lotion base according to the formulation below (Table 12). The capsules were homogeneously distributed in the base and the base became cloudy. Samples were left for 24h at room temperature.

A test sample containing capsules sample 1 was prepared by adding 0.5g of the capsule slurry to 49.5 g of an alcoholic hair setting lotion having the formulation of table 12. The capsules were homogeneously distributed in the base, as observed by optical microscopy. A control sample was created by admixing 0.133g of the free fragrance material no. 1 into 49.867g of the same hair setting. Samples were left for 24h at room temperature and thoroughly mixed just before testing.

Table 12: Alcoholic setting lotion gel formula

| Materials | INCI | % w/w |
|---|---|---|
| Ethanol | Alcohol denaturated | Qsp 100 |
| Luviset CAN | VA / Crotonates / Vinyl neodecanoate copolymer | 6.0 |
| AMP ultra PC 2000 | Aminomethyl propanol | 0.6 |
| Capsule dispersion | | 0.5 |

Test Method: approximately 2.4 grams of the alcoholic setting lotion comprising the capsule dispersion was sprayed onto wet hair swatches from a distance of 10cm. The hair swatch was rubbed gently by hand for 30 sec each then air-dried at ambient temperature. A hair swatch treated with the control sample was treated in the same way. Three hours and 24 hours after application the hair swatches were assessed by two trained perfume evaluators combed 3 times and reassessed.

There was no difference in fragrance intensity between the test sample and the control at any assessment point.

**Example 22: Preparation and evaluation of capsules-containing Eau de toilette**

An Eau de Toilette test sample containing capsules sample 1 was prepared according to the formulation of

table 13A. The capsules were homogeneously distributed in the base, as observed by optical microscopy, and the base became cloudy. A comparative control sample was created according to the formulation of table 13B. Samples were left for 24h at room temperature and thoroughly mixed just before testing.

Table 13A: Test Sample Eau de Toilette

| **Materials** | **INCI** | **% w/w** |
|---|---|---|
| Ethanol | Alcohol denaturated | Qsp 100 |
| Water | Aqua | 14 |
| Capsule Sample 1 | | 5 |

Table 13B: Control Sample Eau de Toilette

| **Materials** | **INCI** | **% w/w** |
|---|---|---|
| Ethanol | Alcohol denaturated | Qsp 100 |
| Water | Aqua | 14.54 |
| fragrance material no.1 | | 1.35 |

Test Method: approximately 0.3 grams of the Eau de Toilette containing capsules was sprayed onto a fragrance blotter from a distance of 10 cm in a fume hood. A blotter of the control sample EdT was prepared in the same way. After 2 minutes, in a different location, the fragrance intensity of the blotters was assessed by two trained perfume evaluators. Then the blotters were rubbed slightly and the perfume intensity was re-assessed.

There was no difference in fragrance intensity between the test samples and the controls.

**Example 23: Preparation and evaluation of capsules-containing body spray aerosol**

A test sample containing capsules sample 1 was prepared by adding 0.5g of the capsule slurry to 49.5 g of a body spray base having the formulation of table 14. The capsules were homogeneously distributed in the base. A control sample was created by admixing 0.133g of the free fragrance material no. 1 into 49.867g of the same body spray base. Samples were left for 24h at room temperature and thoroughly mixed just before testing.

Table 14: body spray formula

| **Materials** | **INCI** | **% w/w** |
|---|---|---|
| Hydrocarbons | Butane / Propane | Qsp 100 |
| Ethanol | Alcohol denaturated | 29 |
| Hydagen cat | Triethyl citrate | 2.5 |
| Cetiol HE | PEG-7 glyceryl cocoate | 1.5 |
| Capsule dispersion | | 1 |

Test Method: approximately 0.2g of body spray aerosol comprising the capsule dispersion were sprayed onto blotter from a distance of 10 cm. After 2 minutes, the blotter was rubbed slightly. Blotters were treated with the control sample in exactly the same way. The perfume intensity assessed by two trained perfume evaluators before and after rubbing.

There was no difference in perfume strength before or after rubbing.

**Example 24: Preparation and evaluation of capsules-containing hair foam**

A test sample containing capsules sample 1 was prepared by adding 0.25g of the capsule slurry to 49.5 g of a hair foam base having the formulation of table 15 and making up to 50g with water. A control sample was created by admixing 0.067g of the free fragrance material no. 1 into 49.933g of the same hair foam. Samples were left for 24h at room temperature and thoroughly mixed just before testing.

Table 15: Hair foam formula

| Materials | Inci | % w/w |
|---|---|---|
| Water | Water | Qsp 100 |
| Ethanol | Alcohol denaturated | 10.0 |
| Butane / Propane | Butane / Propane | 10.0 |
| Luviflex Soft | Acrylates copolymer | 3.0 |
| Xiameter MEM 949 | Amodimethicone / Trideceth-12 / Cetrimonium chloride | 0.86 |
| Celquat H 100 | Polyquaternium-4 | 0.35 |
| Triacetin | Triacetin | 0.3 |
| Tego SLM 20 | Polysorbate-20 | 0.3 |
| D Panthenol 74W | Panthenol | 0.2 |
| Capsule dispersion | | 0.5 |
| AMP ultra PC 2000 | Aminomethyl propanol | pH = 6.7 |

Test Method: 0.4 grams of the hair foam comprising the capsule dispersion were applied onto a wet hair swatch. The hair swatch was massaged by hand for 30 sec then air-dried at ambient temperature. A hair swatch was treated with the control sample in exactly the same way. Three hours and 24 hours after application the hair swatches were combed 3 times. Perfume intensity was assessed by two trained perfume evaluators at the time of use then before and after combing after the set time intervals. There was no difference in fragrance intensity between the test sample and the control.

## Claims

**1.** A microcapsule having an average particle size comprised between 7.5 and 50 microns, said capsule comprising a core and a polymeric shell enclosing said core, wherein:

the core comprises a core material comprising an emulsifiable fragrance, and
the shell comprises in polymerized form a monomer blend comprising:

i) a monomer (I) selected from the group consisting of:

- a monoethylenically unsatured monomer,
- dimethyldiallyl ammonium chloride (DMDAAC), and
- mixtures thereof

and

ii) a monomer (II) which is a polyethylenically unsatured monomer and which comprises one or more monomers selected from the group consisting of divinylbenzene, trivinylbenzene, and a methacrylic ester derived from polyhydric linear or branched $C_2$-$C_{24}$ alcohols or from linear or branched $C_2$-$C_{24}$ polyethylene glycols,

wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.

**2.** A microcapsule having an average particle size comprised between 7.5 and 50 microns, said capsule comprising a core and a polymeric shell enclosing said core, wherein:

the core comprises a core material comprising an emulsifiable fragrance, and
the shell comprises in polymerized form a monomer blend comprising:

i) a monomer (I) selected from the group consisting of:

- a monoethylenically unsatured monomer,
- dimethyldiallyl ammonium chloride (DMDAAC), and
- mixtures thereof

and

ii) a monomer (II) which comprises one or more methacrylic esters derived from polyhydric linear or branched $C_2$-$C_{24}$ alcohols or from linear or branched $C_2$-$C_{24}$ polyethylene glycols and which
A1. contains two or more methacrylate ester groups per monomer, and
B1. has a molecular weight which, once divided by the number of methacrylate ester groups, gives a value of more than 85 and lower than 135,

wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.

**3.** A microcapsule having an average particle size comprised between 7.5 and 50 microns, said capsule comprising a core and a polymeric shell enclosing said core, wherein:

the core comprises a core material comprising an emulsifiable fragrance, and
the shell comprises in polymerized form a monomer blend comprising:

i) a monomer (I) selected from the group consisting of:

- a monoethylenically unsatured monomer,
- dimethyldiallyl ammonium chloride (DMDAAC), and
- mixtures thereof

and

ii) a monomer (II) which is a polyethylenically unsatured monomer,

wherein monomer (II) is such that the microcapsule provides for a fragrance leakage of less than about 70% when tested upon storage for 4 weeks at 40°C according to the method disclosed in example 7, when the microcapsule is prepared according to the procedure disclosed in example 1 and the microcapsule encapsulates a fragrance material no. 2 as defined in example 1, and
wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.

**4.** The microcapsule according to claim 1, wherein said monoethylenically unsatured monomer (I) is selected from the group consisting of:

a) $C_3$-$C_6$ monoethylenically unsatured mono- or poly carboxylic acids,
b) amides of $C_3$-$C_6$ monoethylenically unsatured mono- or poly carboxylic acids;
c) optionally mono- or polysubstituted $C_1$-$C_{24}$ linear or branched alkyl esters of $C_3$-$C_6$ monoethylenically unsatured mono- or poly carboxylic acids,
and
d) optionally mono- or polysubstituted $C_3$-$C_6$ cycloalkyl esters of $C_3$-$C_6$ monoethylenically unsatured mono- or poly carboxylic acids,
wherein optional substituents are selected from the group consisting of:

- OH
- OR,
- C(O)R,
- $NH_2$,
- NHR,
- $NR_2$,

- $NR_3^+$,
- $C_5$-$C_6$ aromatic or heteroaromatic rings, and

$C_3$-$C_{10}$ cyclo- or heterocyclic alkyl, wherein R is $C_1$-$C_4$ alkyl.

**5.** The microcapsule according to any one or more of claims 1 to 4, wherein said monoethylenically unsatured monomer (I) is selected from the group consisting of: methacrylic acid, methyl methacrylate, ethyl methacrylate, isopropyl methacrylate, isobornyl methacrylate and mixtures thereof.

**6.** The microcapsule according to any one or more of claims 1 to 5, wherein said monoethylenically unsatured monomer (I) comprises a combination of methacrylic acid and methyl or ethyl methacrylate.

**7.** The microcapsule according to any one or more of claims 1 to 6, wherein said monomer (II) comprises one or more monomers selected from the group consisting of 1,4-butylene glycol dimethacrylate; 1,3-butylene glycol dimethacrylate; pentaerythritol trimethacrylate; glycerol trimethacrylate; 1,2-propylene glycol dimethacrylate, 1,3-propylene glycol dimethacrylate, ethylene glycol dimethacrylate, diethylene glycol dimethacrylate; glycerol dimethacrylate; trimethylolpropane trimethacrylate; ethoxylated pentaerythritol tetramethacrylate; divinylbenzene, and trivinylbenzene in an amount of at least 10% by weight over the combined weight of all monomers (II) present in the blend.

**8.** The microcapsule according to any one or more of claims 1 to 7, wherein said monomer (II) comprises at least 1,4-butylene glycol dimethacrylate or at least ethylene glycol dimethacrylate.

**9.** The microcapsule according to any one or more of claims 1 to 8 wherein said monomer (II) consists of 1,4-butylene glycol dimethacrylate or ethylene glycol dimethacrylate.

**10.** The microcapsule according to any one or more of claims 1 to 9, wherein said emulsifiable fragrance comprises a combination of at least four distinct emulsifiable fragrances.

**11.** The microcapsule according to any one or more of claims 1 to 10, wherein the core material further comprises a perfumery acceptable solvent.

**12.** The microcapsule according to any one or more of claims 1 to 11 wherein the core-to-shell weight ratio is comprised between 50:1 and 1:1.

**13.** The microcapsule according to any one or more of claims 1 to 12 wherein

- monomer (I) consists of a combination of methacrylic acid 40% by weight, methyl or ethyl methacrylate 16% by weight, and
- monomer (II) consists of 1,4-butane diol dimethacrylate 44% by weight or ethylene glycol dimethacrylate 44 by weight,

wherein percentages are expressed over the combined weight of monomers (I) and (II) in the blend.

**14.** A process for the manufacture of a microcapsule as defined in any one or more of claims 1 to 13, which comprises the following steps:

a) preparing an oil-in-water emulsion having an oil phase and a water phase, said emulsion comprising:

- a polymerization initiator,
- a fragrance material comprising the emulsifiable fragrance,
- the monomer blend,
- an emulsifier, and optionally
- one or more further ingredients to be encapsulated

b) triggering polymerization within the emulsion obtained in step a),
c) letting the polymerization to propagate thereby obtaining microcapsules.

**15.** A water-based dispersion comprising a microcapsule as defined in any one or more of claims 1 to 13.

**16.** A product comprising a microcapsule as defined in any one or more of claims 1 to 13 which is selected from the group consisting of:

- a non-edible consumer goods product,
- a household cleaner or laundry product,
- a personal care product,
- a cosmetic product.

**17.** Use of a polyethylenically unsatured monomer and which comprises one or more monomers selected from the group consisting of divinylbenzene, trivinylbenzene, and a methacrylic ester derived from polyhydric linear or branched $C_2$-$C_{24}$ alcohols or from linear or branched $C_2$-$C_{24}$ polyethylene glycols, to reduce leakage from a microcapsule comprising a core and a polymeric shell enclosing said core, wherein the core comprises a core material comprising an emulsifiable fragrance.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9924525 A
- WO 2005116559 A
- WO 2005105291 A
- US 6849591 B
- US 6951836 B
- WO 2010119020 A
- US 20100286018 A
- EP 1894603 A1
- US 3516941 A
- US 4520142 A
- US 4528226 A
- US 4681806 A
- US 4145184 A
- GB 2073132 A
- WO 9917871 A
- EP 21558474 A
- EP 1572767 A
- EP 2188364 A
- EP 1019478 A
- US 7069658 B
- US 6944952 B
- US 6594904 B
- US 6182365 B
- US 6185822 B
- US 6298558 B
- US 5113585 A
- US 6162423 A
- US 5968286 A
- US 5935561 A
- US 5932203 A
- US 5837661 A
- US 5776443 A
- US 5756436 A
- US 5661118 A
- US 5618523 A
- US 3697644 A
- US 4065398 A
- US 4387040 A
- US 4775582 A
- WO 0207701 A
- WO 2007069214 A
- WO 9516474 A
- WO 9108283 A
- EP 743280 A
- WO 9634938 A
- WO 012351 A
- WO 9928428 A
- US 6335315 B
- US 5674832 A
- US 5759990 A
- US 5877145 A
- US 5574179 A
- US 5929022 A
- US 5916862 A
- US 5731278 A
- US 5470507 A
- US 5466802 A
- US 5460752 A
- US 5458810 A
- US 5011681 A
- US 4421769 A
- US 3755560 A


### Non-patent literature cited in the description


- **S. ARCTANDER.** Perfume Flavors and Chemicals. 1969, vol. I, II
- Allured's Flavor and Fragrance Materials. Allured Publishing Corp,
- **A D MCNAUGHT ; A WILKINSON.** IUPAC Compendium of Chemical Terminology. Blackwell Scientific Publications, 1997
- IUPAC Nomenclature of Organic Chemistry. Blackwell Scientific Publications, 1993
- **A. LEO.** Comprehensive Medicinal Chemistry. Pergamon Press, 1990, vol. 4, 295
- **M ERMAN.** *Cosmetics and Toiletries,* 2005, vol. 120 (5), 105
- MICROENCAPSULATION Methods and Industrial Applications. Marcel Dekker, Inc, 1996
- **MOAD, GRAEME ; SOLOMON, DAVID H.** The Chemistry of Radical Polymerization. Elsevier, 2006
- **BRAUNECKER, WADE A. ; MATYJASZEWSKI, KRZYSZTOF.** Controlled/Living Radical Polymerization: Features, Developments, and Perspectives. *Progress in Polymer Science,* 2007, vol. 32 (1), 93-146
- **BOUTEVIN, BERNARD.** From Telomerization to Living Radical Polymerization. *Journal of Polymer Science Part A: Polymer Chemistry,* 2000, vol. 38 (18), 3235-3243

- **T F TADROS et al.** Emulsion Science and Technology. Wiley-VCH, 2009
- **L HO TAN TAI.** Formulating Detergents and Personal Care Products A guide to Product Development. AOCS Press
- Surfactant Science Series Liquid Detergents. Marcel Dekker Inc, vol. 67
- Harry's Cosmeticology. CHS Press, 2000
- **MCCUTCHEON'S.** Detergents and Emulsifiers. Allured Publishing Corporation, 1986